# EUROPÄISCHE PATENTSCHRIFT

(11) **EP 1 685 134 B1**
(45) Veröffentlichungstag und Bekanntmachung des Hinweises auf die Patenterteilung: **11.02.2009**
(21) Anmeldenummer: 03776835.5
(22) Anmeldetag: 05.11.2003
(51) Int. Cl.: C07D 491/14, A61P 35/00

(54) **NEUE CHELIDONIN-DERIVATE, VERFAHREN ZU IHRER HERSTELLUNG UND IHRE VERWENDUNG ZUR HERSTELLUNG VON PHARMAZEUTISCHEN WIRKSTOFFEN**
NOVEL CHELIDONINE DERIVATIVES, METHODS FOR THE PRODUCTION THEREOF, AND USE THEREOF FOR PRODUCING PHARMACEUTICAL AGENTS
NOUVEAUX DERIVES DE CHELIDONINE, LEUR PROCEDE DE PRODUCTION ET LEUR UTILISATION POUR PRODUIRE DES PRINCIPES ACTIFS PHARMACEUTIQUES

(43) Veröffentlichungstag der Anmeldung: 02.08.2006
(73) Patentinhaber: Salama, Zoser B. nat.rer.Dr., 13465 Berlin (DE)
(72) Erfinder: Salama, Zoser B. nat.rer.Dr., 13465 Berlin (DE)
(74) Vertreter: Boeckh, Tobias
(86) Internationale Anmeldenummer: PCT/DE2003/003702
(87) Internationale Veröffentlichungsnummer: WO 2005/044820

(56) Entgegenhaltungen:
- WO-A-02/092085
- GRYNKIEWICZ G ET AL: "Synthesis and biological activity of O-acyl and O-alkyl chelidonine derivatives" EUROPEAN JOURNAL OF MEDICINAL CHEMISTRY, EDITIONS SCIENTIFIQUE ELSEVIER, PARIS, FR, Bd. 36, Nr. 11-12, November 2001 (2001-11), Seiten 951-960, XP004400915 ISSN: 0223-5234

## Beschreibung

Die Erfindung betrifft neue Chelidonin-Derivate und Verfahren zu ihrer Herstellung; die Erfindung betrifft weiterhin die Verwendung dieser Verbindungen in der Prophylaxe, Therapie, Verlaufskontrolle und in der Nachbehandlung von mit Zellwachstum, Zelldifferenzierung und/oder Zellteilung im Zusammenhang stehenden Krankheiten, insbesondere Tumoren, und einen Kit, der die Chelidonin-Derivate umfasst.

Es ist bekannt, dass Inhaltsstoffe aus verschiedenen Heilpflanzen, wie beispielsweise der Mariendistel, der Mistel, dem Schwarzkümmel, dem Sonnenhut, der Teufelskralle, aber auch dem Schöllkraut (Chelidonium majus) bei verschiedenen Krankheitsbildern eingesetzt werden können. So wirkt das Schöllkraut gegen Warzen und kann gegen verschiedene Gallenerkrankungen eingesetzt werden. Das Schöllkraut enthält zirka 30 Alkaloide mit einem Gesamtgehalt von zirka 0,1 bis 1 Gew.-%, darunter unter anderem Chelelerythrin, Sanguinhrin, Berberin und Chelidonin. Es ist bekannt, dass Chelidonin krampflösend und schmerzstillend wirkt. Weiterhin ist bekannt, dass der gelbe Milchsaft der frischen Pflanze aufgrund des Gehalts an Chelidonin die Zellteilung che, Chelidonin-Derivate bereitzustellen, die spezifisch gegen Tumorzellen wirken, jedoch nur geringe bzw. gar keinen Effekt auf nicht entartete, das heißt nicht tumorige Zellen, zeigen. So beschreiben Jalilian et al. (2001) die Synthese-Chelidonin-Derivate, die möglicherweise als antitumorale Wirkstoffe eingesetzt werden können. Bei den Derivaten handelt es sich um Fluorobenzoate, die so markiert sind, dass mit ihnen Untersuchungen der Wechselwirkung mit mikrotubulären Strukturen durchgeführt werden können. Grynkiewicz et al. (2001) beschreiben verschiedene Chelidonin-Derivate, die Wirkungen auf das Zentralnervensystem haben. Die offenbarten Derivate zeigen insbesondere einen antiserotoninergen Effekt, der für die Muttersubstanz Chelidonin nicht offenbart ist.

Ein weiteres Chelidonin-Derivat ist Ukrain, das als trimäre Verbindung aus Schöllkrautalkaloiden mit Thiophosphorsäuretriazit in der Krebstherapie angewendet wird. Es konnte gezeigt werden, dass in Zellkulturen und in Tierversuchen eine antitumoröse Wirksamkeit von Ukrain nachgewiesen werden kann. Weiterhin gibt es einzelne Offenbarungen, aus denen hervorgeht, dass Ukrain im humanen Bereich bei Prostatakarzinomen, beim kolorektalen Karzinom und bei Brustkrebs eine therapeutische Wirkung zeigt. Bisher konnte jedoch nicht gezeigt werden, welche Wirksubstanzen innerhalb der unterschiedlichen Chargen von Ukrain für die beschriebene antitumorale Wirkung verantwortlich sind. Keines der verwendeten Mittel besitzt derzeit eine offizielle Zulassung in EU-Mitgliedsstaaten.

Neben dem Zweifel an der Struktur der Substanzen wie auch an der Zusammensetzung fehlt daher für die meisten der verwendeten Substanzen ein Wirkungsnachweis (Hopf, 2002).

Aufgabe der Erfindung war es daher, weitere Chelidonin-Derivate bereitzustellen, die strukturell eindeutig bestimmt werden können und für die eine antitumorale Wirksamkeit ursächlich gezeigt werden kann.

Die Erfindung löst diese erfindungsgemäße Aufgabe durch die Bereitstellung von neuen Chelidonin-Derivaten mit einer antitumoralen Wirkung ausgewählt aus der Gruppe umfassend Chelidonin-Acetat, Trifluoressigsäure-Chelidonylester, Chelidonyl-trichlormethyl-kohlensäureester, Bernsteinsäure-chelidoninyl-methylester, Chelidonyl-ethyl-oxalsäurediester, N-(3-Trifluormethylphenyl)-chelidonyl-urethan, Chelidonyl-phenylalanylester, Chelidonyl-prolylester und/oder Chelidonyl-alanylester.

Überraschenderweise konnte gezeigt werden, dass die synthetisierten Chelidonin-Derivate eine antitumorale Wirksamkeit zeigen.

Die Erfindung betrifft auch pharmazeutische Mittel, die die erfindungsgemäßen Chelidonin-Derivate gegebenenfalls zusammen mit einem pharmazeutisch verträglichen Träger, einem Adjuvants und/oder einen Vehikel umfassen.

Im Folgenden werden die Begriffe Chelidonin-Derivate, erfindungsgemäße Verbindungen und pharmazeutische Mittel synonym verwendet, das heißt, sofern es Ausführungen zu

In der WO 021092085 wird ein Chelidonin-Acetat offenbart, welches als Antiarrhythmikum eingesetzt wird. Die Druckschrift offenbart ganz allgemein pharmazeutische Zusammensetzungen, die Chelidonin oder Derivate hiervon umfassen, die nach Ansicht der Autoren der WO 021092085 Einfluss auf Kaliumkanäle haben, die für den Aufbau und Regulation des Aktionspotentials an Herzmuskeln verantwortlich sind. Die in der WO 0210920858 offenbarten Chelidonin-Derivate, wie Chelidonin-Acetat, werden als exzellente Kalziumkanalblocker beschrieben. Die Autoren regen aber an keiner Stelle der WO 02/092085 an die von ihnen bereitgestellten Verbindungen in der Tumortherapie einzusetzen.

Grynkiewicz et al., 2001, offenbaren 11 Derivate von Chelidonin und untersuchen ihren Einfluss auf die Bewegungskoordination von Mäusen, wobei die Wirkung der offenbarten Verbindungen auf das zentrale Nervensystem untersucht wird. Wie auch in der WO 02/092085 wird auch in Grynkiewicz et al, 2001 ein Chelidonin-Acetat beschrieben. Grynkiewicz et al. offenbaren aber nicht oder legen nahe, diese Verbindung im Zusammenhang mit Tumorerkrankungen einzusetzen.

Ein Fachmann würde die WO 02/092085 und Grynkiewicz et al. aufgrund ihrer unterschiedlichen Aufgaben, die auch von der Aufgabe der erfindungsgemäßen Lehre verschieden ist, nicht heranziehen, um antitumorale Derivate bereitzustellen. Aber auch wenn man die beiden Druckschriften miteinander kombinierte, würde diese Kombination kein Sprungbrett darstellen, um zur erfindungsgemäßen Lehre zu gelangen. tel können in einer therapeutischen Menge mit einem Organismus in Kontakt gebracht werden.

Der Ausdruck therapeutische Menge bezeichnet wie hierin verwendet eine Menge, die Symptome einer Störung oder responsiven, pathologisch physiologischen Kondition verhindert oder verbessert. In bestimmten Ausführungsformen der vorliegenden Erfindung ist die verabreichte Menge ausreichend, einen Tumor im Wachstum zu hemmen, die im Wesentlichen die Ausbreitung eines Tumors, die Tumor-Angiogenese, die Tumor-Invasion und/oder die Tumor-Metastasierung im Rezipienten verhindert oder hemmt. Die Erfindung betrifft demgemäß pharmazeutische Mittel oder Arzneimittel, die die erfindungsgemäßen Verbindungen umfassen, gegebenenfalls zusammen mit pharmazeutischen Hilfsstoffen.

Die an einem Gesunden im Falle der Prophylaxe bzw. an einem Patienten im Falle der Therapie zu verwendende Menge an erfindungsgemäßen Verbindungen wird formuliert und die Dosis gemäß üblicher medizinischer Praxis festgesetzt, wobei die zu behandelnde Störung, der Zustand des einzelnen Patienten, die Verabreichungsstelle, das Verabreichungsverfahren und andere, den behandelnden Ärzten bekannte Faktoren berücksichtigt werden. In ähnlicher Weise hängt die Dosis der verabreichten erfindungsgemäßen Verbindungen von den Eigenschaften des Tumors ab, von der in vivo Halbwertszeit erfindungsgemäßen Verbindungen im Plasma wie auch von der Konzentration der erfindungsgemäßen Verbindungen in der Formulierung, dem Verabreichungsweg, der Stelle und der Rate der Dosierung, der klinischen Toleranz des jeweiligen Individuums (Mensch und Tier), der pathologischen Affektion des Patienten und dergleichen, wie es Ärzten bzw. anderen Fachleuten bekannt ist. Im Allgemeinen werden Dosierungen von etwa 0,1 bis 1000 mg pro Individuum und Verabreichung bevorzugt; besonders bevorzugt ist eine Dosierung von 10 Plasma wie auch von der Konzentration der erfindungsgemäßen Verbindungen in der Formulierung, dem Verabreichungsweg, der Stelle und der Rate der Dosierung, der klinischen Toleranz des jeweiligen Individuums (Mensch und Tier), der pathologischen Affektion des Patienten und dergleichen, wie es Ärzten bzw. anderen Fachleuten bekannt ist. Im Allgemeinen werden Dosierungen von etwa 0,1 bis 1000 mg pro Individuum und Verabreichung bevorzugt; besonders bevorzugt ist eine Dosierung von 10 bis 500 mg, ganz besonders bevorzugt von 200 bis 400 mg, insbesondere von 300 mg. Es können während einer Abfolge aufeinander folgender Verabreichungen auch unterschiedliche. Dosierungen eingesetzt werden.

Es ist zum Beispiel vorgesehen, dass Injektionen (intramuskulär oder subkutan oder in die Blutgefäße) ein Weg für die therapeutische Verabreichung der erfindungsgemäßen Verbindungen, beispielsweise der eingekapselten oder an Träger gebundenen erfindungsgemäßen Verbindungen, sind, obgleich die Zufuhr als Aerosol, über Katheter oder chirurgische Schläuche auch angewendet werden kann. Andere bevorzugte Wege umfassen Suspensionen, Tabletten, Kapseln und dergleichen für die orale Verabreichung, im Handel erhältliche Vernebler für flüssige Formulierungen und Inhalationen von lyophilisierten oder aerolysierten Verbindungen und Suppositorien für rektale oder vaginale Verabreichung. Flüssige Formulierungen können Lösungen, ein Sirup, flüssige Mischungen, Suspensionen, Emulsionen und/oder Infusionen sein. Die Eignung der gewählten Parameter, zum Beispiel Dosis, Schema, Adjuvanzwahl und dergleichen, kann durch Entnahme von Serum-Aliquoten aus dem Patienten - das heißt dem Mensch oder dem Tier - und Tes-Exzipienten vermengt werden, um für eine stabilisierende Wirkung vor der Behandlung beispielsweise mit einem Lösungsmittel zu sorgen. Eine wässrige Lösung einer erfindungsgemäßen Verbindung kann eine erfindungsgemäße Verbindung in Suspension oder eine Lösung sein.

Die erfindungsgemäße Verbindung kann in einer Lösung mit einem Konservierungsmittel eingebracht sein. Beispiele für geeignete Konservierungsmittel der Suspension bzw. Lösungen umfassen Phenol, Benzylalkohol, m-Kresol, Metylparaben, Propylparaben, Benzalkoniumchlorid und Benzethoniumchlorid. Im Allgemeinen können die Formulierungen der erfindungsgemäßen Verbindungen Komponenten in Mengen enthalten, die der Herstellung stabiler Formen nicht abträglich sind und Mengen, die für wirksame, sichere pharmazeutische Verabreichungen geeignet sind. Beispielsweise können andere pharmazeutisch annehmbare, dem Fachkundigen bekannte Exzipienten einen Teil der erfindungsgemäßen Verbindungen oder Formulierungen bilden. Diese umfassen beispielsweise Salze, verschiedene Füller, zusätzliche Pufferagenzien, Chelatbildner, Antioxydanzien, Co-Lösungsmittel und dergleichen.

In einer bevorzugten Ausführungsform der Erfindung sind die erfindungsgemäßen Verbindungen mit Liposomen, Siosomen und/oder Niosomen assoziiert.

Dies kann beispielsweise so erfolgen, dass die erfindungsgemäße Verbindung in einem Liposom eingeschlossen oder auf der Liposomenoberfläche verankert vorliegt. Dem Fachmann ist bekannt, dass künstliche bzw. natürliche Membranen von Liposomen eine immunstimulierende Wirkung haben können, insbesondere dann, wenn die Komponenten auf die Oberfläche der Liposomen gekoppelt werden oder im Inneren der Liposomen eingeschlossen sind oder einfach nur mit den Liposomen zusammen vermischt werden. Derartige Formulierungen von Liposomen können parentral appliziert werden. Es ist möglich, derartige Formulierungen mit bekannten Methoden, wie zum Beispiel einem Spray, nasal auf die Schleimhäute der Nasenhöhlen zu applizieren. Eine mit dem Spray vorgenommene therapeutische Behandlung ist bevorzugt für die Behandlung von Lungenkrebs oder Hals-Nasen-Ohren Tumoren geeignet. Insbesondere bei der nasalen Verabreichung muss die erfindungsgemäße Verbindung in einem solchen Zustand auf die Schleimhaut aufgetragen werden, dass sie in der Lage ist, die Schleimhaut zu durchdringen oder durch diese absorbiert zu werden. Daher muss das Vesikel mit dem Schleim biokompatibel sein und ein gewisses Maß an Hydrophilie aufweisen. Dem Fachmann sind derartige Strukturen beispielsweise aus der EP 0682528 bekannt, deren Lehre mit in den Offenbarungsgehalt der Erfindung aufgenommen ist. Die liposomale Zusammensetzung kann einen oder mehrere zusätzliche pharmazeutische Träger umfassen, die ausgewählt sind aus oberflächenaktiven Stoffen und Absorptionsförderern wie zum Beispiel Polyoxyethylenalkoholethern, Gallensalzen und deren Derivaten, Fusidinsäure und deren Derivaten, Oleinsäure, Lecithin, Lysolecithinen, Tween® 21 bis 85, usw., wasserabsorbierenden Polymeren wie zum Beispiel Glycofurol, Polyethylenglycol 200 bis 7500, Polyvinylpyrrolidon, Propylenglycol oder Polyacrylsäure, Gelatine, Cellulose und Derivaten usw.; Substanzen, welche den enzymatischen Abbau hemmen, wie zum Beispiel Aprotinin usw.; organischen Lösungsmitteln wie zum Beispiel Alkoholen, zum Beispiel Ethanol, Glycerol, Benzylalkohol usw.; oder Ethylacetat usw.; hydrophoben Mitteln wie zum Beispiel pflanzlichem Öl, Sojabohnenöl, Erdnussöl, Kokosnussöl, Maisöl, Olivenöl, Sonnenblumenöl, "Miglyolen" oder deren Mischungen usw.; pH-regulierenden Mitteln wie zum Beispiel Salpetersäure, Phosphorsäure, Essigsäure, Citraten usw.; Konservierungsmitteln und den osmotischen Druck regulierenden Mitteln wie zum Beispiel Glycerol, Natriumchlorid, Methylparaoxybenzoat, Benzoesäure usw.; Liposomen- und/oder Emulsionsformulierungen wie zum Beispiel Lecithinen usw.; mikroverkapselten Formulierungen; Treibmitteln wie zum Beispiel Butan.

Bevorzugt ist es in einer weiteren Ausführungsform der Erfindung, dass die erfindungsgemäßen Verbindungen gegebenenfalls miteinander assoziiert oder mit einem Träger verbunden in Liposomen eingeschlossen sind, wobei der Einschluss in Liposomen im Sinne der Erfindung nicht zwingend bedeuten muss, dass die erfindungsgemäßen Verbindungen im Inneren der Liposomen vorliegen, ein Einschluss im Sinne der Erfindung kann auch bedeuten, dass die erfindungsgemäßen Verbindungen mit der Membran der Liposomen assoziiert sind, beispielsweise so, dass diese auf der äußeren Membran verankert sind. Eine solche Darstellung der erfindungsgemäßen Verbindungen in oder auf den Liposomen ist vorteilhaft, wenn der Fachmann die Liposomen so auswählt, dass sie eine immunstimulierende Wirkung haben. Dem Fachmann sind aus der DE 198 51 282 verschiedene Möglichkeiten bekannt, die immunstimulierende Wirkung von Liposomen zu modifizieren. Bei den Lipiden kann es sich um einfache Lipide handeln, wie beispielsweise Ester und Amide oder um komplexe Lipide wie zum Beispiel um Glycolipide wie Cerebroside oder Ganglioside, um Sphingolipide oder um Phosphorlipide.

Bei den Trägern, die Bestandteil von Arzneimitteln sein können - die die erfindungsgemäßen Verbindungen umfassen -, kann es sich im Sinne der Erfindung um Proteine handeln, die aufgrund ihres immunogenen Verhaltens die Antikörperantwort stimulieren, aber auch um den Fachmann bekannte pharmazeutische Hilfsstoffe wie zum Beispiel QS21, GPI-0100 oder andere Saponine, Wasser-Öl-Emulsionen wie beispielsweise Montanide, Polylysin, Polyagenin-Verbindungen oder andere, wie zum Beispiel phosphat-gepufferte Kochsalzlösung, Wasser, verschiedene Arten von Detergenzien, sterile Lösungen und dergleichen mehr.

Ein pharmazeutisches Mittel im Sinne der Erfindung ist jedes Mittel im Bereich der Medizin, welches in der Prophylaxe, Diagnose, Therapie, Verlaufskontrolle oder Nachbehandlung von Patienten eingesetzt werden kann, die einen Tumor so umfassen, dass sich zumindest zeitweise eine pathogene Modifikation des Gesamtzustandes bzw. des Zustandes einzelner Teile des Organismus etablieren konnte. So ist es beispielsweise möglich, dass das pharmazeutische Mittel im Sinne der Erfindung eine Vakzine oder ein Therapeutikum ist. Das pharmazeutische Mittel im Sinne der Erfindung kann neben den erfindungsgemäßen Verbindungen beispielsweise ein akzeptables Salz oder Komponenten dieser umfassen. Hierbei kann es sich beispielsweise um Salze anorganischer Säuren handeln wie zum Beispiel der Phosphorsäure bzw. um Salze organischer Säuren.

Weiterhin ist es möglich, dass die Salze frei von Carboxylgruppen sind und von anorganischen Basen abgeleitet wurden wie zum Beispiel Natrium, Kalium, Ammonium, Calcium oder Eisenhydroxyde oder auch von organischen Basen wie Isopropylamin, Trimethylamin, 2-Ethylaminoethanol, Histidin und andere. Beispiele für flüssige Träger sind sterile wässrige Lösungen, die keine weiteren Materialien oder aktiven Ingredienzien umfassen und beispielsweise Wasser oder solche, die einen Puffer wie zum Beispiel Natriumphosphat mit einem physiologischen pH umfassen oder eine physiologische Salzlösung bzw. beides, wie zum Beispiel phosphat-gepufferte Natriumchloridlösung. Weitere flüssige Träger können mehr als nur ein Puffersalz, wie zum Beispiel Natrium- und Kaliumchlorid, Dextrose, Propylenglycol, Polyethylenglycol oder andere umfassen. Flüssige Züsammensetzungen der pharmazeutischen Mittel können zusätzlich eine flüssige Phase, jedoch unter dem Ausschluss von Wasser, umfassen. Beispiele solcher zusätzlichen flüssigen Phasen sind Glycerin, Pflanzenöle, organische Ester oder Wasser-Öl-Emulsionen. Die pharmazeutische Zusammensetzung bzw. das pharmazeutische Mittel enthält typischerweise einen Gehalt von mindestens 0,1 Gew% der erfindungsgemäßen Verbindungen bezogen auf die gesamte pharmazeutische Zusammensetzung. Die jeweilige Dosis bzw. der Dosisbereich für die Gabe des erfindungsgemäßen pharmazeutischen Mittels ist groß genug, um den gewünschten prophylaktischen oder therapeutischen Effekt der Bildung von neutralisierenden Antikörpern zu erreichen. Hierbei sollte die Dosis nicht so gewählt werden, dass unerwünschte Nebeneffekte dominieren. Im Allgemeinen wird die Dosis mit dem Alter, der Konstitution, dem Geschlecht des Patienten variieren sowie selbstverständlich auch in Bezug auf die Schwere der Erkrankung. Die individuelle Dosis kann sowohl in Bezug auf die primäre Erkrankung als auch in Bezug auf Eintreten zusätzlicher Komplikationen eingestellt werden. Die exakte Dosis ist durch einen Fachmann mit bekannten Mitteln und Methoden feststellbar, beispielsweise durch die Feststellung des Tumorwachstums in Abhängigkeit der Dosis bzw. in Abhängigkeit des Applikationsschemas oder der pharmazeutischen Träger und ähnlichem. Die Dosis kann hierbei je nach Patient individuell gewählt werden. Beispielsweise kann eine vom Patienten noch tolerierte Dosis des pharmazeutischen Mittels eine solche sein, deren Bereich im Plasma oder in einzelnen Organen lokal im Bereich von 0,1 bis 10000 µM liegt, bevorzugt zwischen 1 und 100 µM. Alternativ kann die Dosis auch in Bezug auf das Körpergewicht des Patienten bezogen berechnet werden. In einem solchen Fall wäre beispielsweise eine typische Dosis des pharmazeutischen Mittels in einem Bereich zwischen 0,1 µg bis 100 µg per kg Körpergewicht einzustellen, bevorzugt zwischen 1 und 50 µg/kg. Weiterhin ist es jedoch auch möglich, die Dosis nicht in Bezug auf den gesamten Patienten, sondern in Bezug auf einzelne Organe zu bestimmen. Dies wäre beispielsweise dann der Fall, wenn das erfindungsgemäße pharmazeutische Mittel beispielsweise in einem Biopolymer, eingebracht in den jeweiligen Patienten, in der Nähe bestimmter Organe mittels einer Operation platziert wird. Dem Fachmann sind mehrere Biopolymere bekannt, die Peptide oder rekombinante Proteine in einer gewünschten Art und Weise freisetzen können. Ein solches Gel kann beispielsweise 1 bis 1000 µg der erfindungsgemäßen Zusammensetzungen, bzw. des pharmazeutischen Mittels pro ml Gelkomposition beinhalten, bevorzugt zwischen 5 bis 500 µg/ml und besonders bevorzugt zwischen 10 und 100 µg/ml. In solch einem Fall wird das therapeutische Mittel als feste, gel-artige oder als flüssige Komposition verabreicht.

In einer weiteren bevorzugten Ausführungsform der Erfindung sind die Träger ausgewählt aus der Gruppe umfassend Füllmittel, Streckmittel, Bindemittel, Feuchthaltemittel, Sprengmittel, Lösungsverzogerer, Resorptionsbeschleuniger, Netzmittel, Adsorbtionsmittel, und/oder Gleitmittel.

Hierbei handelt es sich bei den Füll- und Streckmitteln bevorzugt um Stärken, Milchzucker, Rohrzucker, Glukose, Mannit und Kieselsäure, bei dem Bindemittel, bevorzugt Carbocymethylcellulose, Alginate, Gelatine, Polyvinylpyrrolidon, bei dem Feuchthaltemittel bevorzugt um Glycerin, bei dem Sprengmittel bevorzugt um Agar-Agar, Calciumcarbonat und Natriumcarbonat, bei dem Lösungsverzögerer vordem Gleitmittel bevorzugt um Talkum, Calcium- und Magnesiumstearat und feste Polythylenglykole oder Gemische der aufgeführten Stoffe.

In einer weiteren bevorzugten Ausführungsform_der Erfindung werden die erfindungsgemäßen Verbindungen als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und/oder in dieser Form angewendet. Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Arzneimittel dieser Erfindung können bevorzugt zur oralen Verabreichung in einer beliebigen oral verträglichen Dosierungsform verwendet werden, die Kapsel, Tabletten und wässrige Suspensionen und Lösungen einschließt, aber nicht darauf beschränkt ist. Im Fall von Tabletten zur oralen Verwendung schließen Träger, die häufig verwendet werden, Lactose und Maisstärke ein. Gleitmittel, wie Magnesiumstearat, werden auch typischerweise zugesetzt. Zur oralen Verabreichung in Kapselform schließen verwendbare Verdünnungsmittel Lactose und getrocknete Maisstärke ein. Wenn wässrige Suspensionen oral verabreicht werden, wird der Wirkstoff mit Emulgier- und Suspendiermitteln kombiniert. Falls gewünscht, können bestimmte Süßmittel und/oder Geschmacksstoffe und/oder Farbmittel zugesetzt werden.

Der oder die Wirkstoffe - das heißt die erfindungsgemäßen Verbindungen - können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄-Alkohol mit C₁₆-Fettsäure) oder Gemische dieser Stoffe).

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydofurfurylalkohol, Polyethylenglycole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer sterilen injizierbaren Zubereitung, zum Beispiel als sterile injizierbare wässrige oder ölige Suspension, vorliegen. Diese Suspension kann auch im Fachgebiet bekannten Verfahren unter Verwendung geeigneter Dispergier- oder Netzmittel (wie zum Beispiel Tween 80) und Suspendiermittel formuliert werden. Die sterile injizierbare Zubereitung kann auch eine sterile injizierbare Lösung oder Suspension.in einem ungiftigen parenteral verträglichen Verdünnungs- oder Lösungsmittel, zum Beispiel als Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung. Außerdem werden üblicherweise sterile, nichtflüchtige Öle als Lösungsmittel oder Suspendiermediurn verwendet. Zu diesem Zweck kann ein beliebiges mildes nichtflüchtiges Öl einschließlich synthetischer Mono- oder Diglyceride verwendet werden. Fettsäuren, wie Ölsäure und ihre Glyceridderivate sind bei der Herstellung von Injektionsmitteln verwendbar, wie es natürliche pharmazeutisch verträgliche Öle, wie Olivenöl oder Rizinusöl, insbesondere in ihren polyoxyethylierten Formen sind. Diese Öllösungen oder Suspensionen können auch einen langkettigen Alkohol oder einen ähnlichen Alkohol enthalten als Verdünnungs- oder Dispergiermittel.

Die genannten Formulierungsformen können auch Färbemittel, Konservierungsstöffe sowie geruchs- und geschmacksverbesserte Zusätze, zum Beispiel Pfefferminzöl und Eukalyptusöl und Süßmittel, zum Beispiel Saccharin, enthalten. Die erfindungsgemäßen Verbindungen sollen in den aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die aufgeführten pharmazeutischen Zubereitungen können außer den erfindungsgemäßen Verbindungen auch weitere pharmazeutische Wirkstoffe enthalten. Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie.zum Beispiel Kunststoffe, (Kunststoffketten zur lokalen Therapie), Kollagen oder Knochenzement eingearbeitet werden.

In einer weiteren bevorzugten Ausführungsform der Erfindung sind die erfindungsgemäßen Verbindungen in einer Konzentration von 0,1 bis 99,5, bevorzugt von 0,5 bis 95, besonders bevorzugt von 20 bis 80 Gel.-% in einer Zubereitung eingebracht. Das heißt, die erfindungsgemäßen Verbindungen sind in den oben aufgeführten pharmazeutischen Zubereitungen, zum Beispiel Tabletten, Pillen, Granulaten und anderen, vorzugsweise in einer Konzentration von 0,1 bis 99,5 Gew.-% der Gesamtmischung vorhanden. Die Wirkstoffmenge, das heißt die Menge einer erfindungsgemäßen Verbindung, die mit den Trägermaterialien kombiniert wird, um eine einzige. Dosierungsform zu erzeugen, wird von dem Fachmann in Abhängigkeit von dem zu behandelnden Wirt, vom zu behandelnden Tumor und der besonderen Verabreichungsart variieren können. Nach Besserung des Zustandes eines Wirts bzw. eines Patienten kann der Anteil der wirksamen Verbindung in der Zubereitung so geändert werden, dass eine Erhaltungsdosis vorliegt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Verbindungen, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäßen Verwindungen mit einem. Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass der Tumor möglichst effektiv bekämpft wird bzw. der Ausbruch einer solchen Krankheit in einer prophylaktischen Gabe verhindert wird. Die Konzentration und die Art der Applikation kann vom Fachmann durch Routineversuche eruiert werden. Bevorzugte Applikationen der erfindungsgemäßen Verbindungen sind die orale Applikation in Form von Pulver, Tabletten, Saft, Tropfen, Kapseln oder ähnlichem, die rektale Applikation in Form von Zäpfchen, Lösungen und ähnlichem, parenteral in Form von Injektionen, Infusionen und Lösungen, Inhalation von Dämpfen, Aerosolen und Stäuben und Pflastern sowie lokal in Form von Salben, Pflastern, Umschlägen, Spülungen und ähnlichem. Bevorzugt erfolgt das In-Kontakt-Bringen der erfindungsgemäßen Verbindungen prophylaktisch oder therapeutisch. Bei einer prophylaktischen Gabe soll die Bildung den genannten Tumoren zumindest dergestalt verhindert werden, dass eine weitere Vermehrung dieser stark vermindert wird oder dass Tumoren nahezu vollständig eliminiert werden. Bei einem therapeutischen In-Kontakt-Bringen liegt bereits eine Tumorerkrankung des Patienten vor und die bereits im Körper vorhandenen Tumoren sollen entweder abgetötet oder in ihrer Vermehrung gehemmt werden. Weitere hierfür bevorzugte Applikationsformen sind beispielsweise die subkutane, die sublinguale, die intravenöse, die intramuskuläre, die intraperitoneale und/oder die topische.

Neben den bereits ausgeführten Konzentrationen bei der Anwendung der erfindungsgemäßen Verbindungen können in einer bevorzugten Ausführungsform die Verbindungen weiterhin in einer Gesamtmenge von 0,05 bis 500 mg/kg Körpergewicht je 24 Stunden eingesetzt werden, bevorzugt von 5 bis 100 mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern. Die verabreichte Menge ist ausreichend, um das Tumorwachstum zu hemmen.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,05 bis 500 mg/kg Körpergewicht kann einmalig oder mehrfach angewendet werden, um die gewünschten Ergebnisse zu erhalten. Die Dosishöhen pro Tag sind bei der Vorbeugung und bei der Behandlung einer Tumorerkrankung einschließlich einer Infektion anwendbar, beispielsweise bei Infektionen, die einen Tumor verursachen bzw. mit verursachen können, wie zum Beispiel eine Hepatitis-, insbesondere eine Hepatitis B-Infektion. Typischerweise werden insbesondere pharmazeutis chen Mittel zur etwa 1- bis 7-maligen Verabreichung pro Tag oder alternativ oder zusätzlich als kontinuierliche Infusion verwendet. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Targetsdosis auf 2 bis 5 Applikationen zu verteilen, wobei bei jeder Applikation 1 bis 2 Tabletten mit einem Wirkstoffgehalt von 0,05 bis 500 mg/kg Körpergewicht verabreicht werden. Selbstverständlich ist es möglich, den Wirkstoffgehalt auch höher zu wählen, beispielsweise bis zu einer Konzentration bis 5000 mg/kg. Beispielsweise können Tabletten auch retardiert sein, wodurch sich die Anzahl der Applikationen pro Tag auf 1 bis 3 vermindert. Der Wirkstoffgehalt der retardierten Tabletten kann 3 bis 3000 mg betragen. Wenn der Wirkstoff - wie ausgeführt - durch eine Injektion verabreicht wird, ist es bevorzugt, 1- bis 8-mal pro Tag bzw. durch Dauerinfusion den Wirt mit den erfindungsgemäßen Verbindungen in Kontakt zu bringen, wobei Mengen von 4 bis 4000 mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens leicht erfolgen.

In einer weiteren besonders bevorzugten Ausführungsform der Erfindung werden die erfindungsgemäßen Verbindungen in einer Einzelgabe von 1 bis 80, insbesondere von 3 bis 30 mg/kg Körpergewicht eingesetzt. Wie auch die Gesamtmenge pro Tag kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden. Eine Einzeldosis enthält vorzugsweise die Menge Wirkstoffe die bei einer Applikation verabreicht wird, und die gewöhnlich einer ganzen, einer halben Tagesdosis oder einem Drittel oder einem Viertel einer Tagesdosis entspricht. Die Dosierungseinheiten können demgemäß bevorzugt 1, 2, 3 oder 4 oder mehrere Einzeldosen oder 0,5, 0,3 oder 0,25 einer Einzeldosis enthalten. Bevorzugt wird die Tagesdosis der erfindungsgemäßen Verbindungen auf 2 bis 10 Applikationen verteilt, bevorzugt auf 2 bis 7, besonders bevorzugt auf 3 bis 5 Applikationen. Selbstverständlich ist auch eine Dauerinfusion der erfindungsgemäßen Mittel möglich.

In einer besonders bevorzugten Ausführungsform der Erfindung werden bei jeder oralen Applikation der erfindungsgemäßen Verbindungen 1 bis 10 Tabletten oder Kapseln gegeben, bevorzugt 4 bis 8 Kapseln oder Tabletten und ganz besonders bevorzugt 6 Kapseln oder Tabletten. Die erfindungsgemäßen Tabletten oder Kapseln können mit dem Fachmann bekannten Überzügen und Hüllen versehen sein und auch so zusammengesetzt werden, dass sie den oder die Wirkstoffe nur bei bevorzugten, in einem bestimmten Teil des Wirts freigeben.

In einer weiteren bevorzugten Ausführungsform der Erfindung können die erfindungsgemäßen Verbindungen mit mindestens einem anderen bekannten pharmazeutischen Mittel eingesetzt werden. Das heißt, die erfindungsgemäßen Verbindungen können in einer prophylaktischen oder therapeutischen Kombination in Verbindung mit den bekannten Arzneimitteln eingesetzt werden. Diese Kombinationen können gemeinsam, zum Beispiel in einer einheitlichen pharmazeutischen Formulierung verabreicht werden, oder getrennt, zum Beispiel in Form einer Kombination aus Tabletten, Injektion oder anderen Medikamenten, die zu gleichen oder zu unterschiedlichen Zeiten verabreicht werden, mit dem Ziel, die gewünschte prophylaktische oder therapeutische Wirkung zu erzielen. Bei diesen bekannten Mitteln kann es sich um Mittel handeln, die die Wirkung der erfindungsgemäßen Verbindungen steigern.

Selbstverständlich ist es auch möglich, die erfindungsgemäßen Verbindungen, insbesondere die pharmazeutischen Mittel, allein oder' zusammen mit anderen Mitteln in einer Therapie, beispielsweise in einer Kombinationstherapie, als regionale Therapie einzusetzen; dies kann beispielsweise bei einem Lebertumor bevorzugt sein.

Dem Fachmann ist bekannt, dass es vorteilhaft sein kann, bei bestimmten Tumorerkrankungen die Konzentration von Glutation durch Oxidantien und/oder Adduktbildner herabzusetzen. Hierfür kann es bevorzugt sein, die Konzentration von Platinkomplexen als Chemotherapeutikum bzw. der erfindungsgemäßen Verbindungen zu erhöhen.

Typischerweise gibt es ein optimales Verhältnis der erfindungsgemäßen Verbindung(en) untereinander und/oder mit anderen therapeutischen oder wirkungssteigernden Mitteln (wie Transportinhibitoren und/oder -aktivatoren, Stoffwechselinhibitoren, Inhibitoren oder Aktivatoren für die Nierenausscheidung oder Glukuronidation, wie Probenecid, Acetaminophen, Aspirin, Lorazepam, Cimetidin, Ranitidin, Colifibrat, Indomethacin, Ketoprofen, Naproxen etc.) in dem die Wirkstoffe in einem optimalen Verhältnis vorliegen: Ein optimales Verhältnis ist als das Verhältnis definiert, bei dem die erfindungsgemäße(n) Verbindung(en) mit einem anderen therapeutischen Mittel oder Mitteln so ist, dass die therapeutische Gesamtwirkung größer ist als die Summe der Wirkungen der einzelnen therapeutischen Mittel. Das optimale Verhältnis findet man üblicherweise, wenn die Mittel im Verhältnis von 10:1 bis 1:10, 20:1 bis 1:20, 100:1 bis 1:100 und 500:1 bis 1:500 vorliegen. Gelegentlich wird eine verschwindend geringe Menge eines therapeutischen Mittels genügen, um die Wirkung eines oder mehrerer anderer Mittel zu steigern. Zusätzlich ist die Verwendung der erfindungsgemäßen Verbindungen in Kombinationen besonders nützlich, um das Risiko der Resistenzentwicklung herabzusetzen und/oder die therapeutische Wirksamkeit zu erhöhen. Selbstverständlich können die erfindungsgemäßen Verbindungen in Kombination mit anderen bekannten anti-Tumor Mitteln verwendet werden. Dem Fachmann sind derartige Mittel bekannt. Die erfindungsgemäßen Verbindungen können demgemäß mit allen herkömmlichen Mitteln, insbesondere anderen Arzneimitteln, verabreicht werden, die für die Verwendung im Zusammenhang insbesondere mit Tumor-Arzneimitteln verfügbar sind, entweder als einzelne Arzneimittel oder in Kombination von Arzneimitteln. Sie können allein verabreicht werden oder in Kombination mit diesen.

Bevorzugt ist es, dass die erfindungsgemäßen Verbindungen mit den anderen bekannten pharmazeutischen Mitteln im Verhältnis von etwa 0,005 zu 1 verabreicht werden. Bevorzugt ist es, die erfindungsgemäßen Verbindungen mit insbesondere virushemmenden Mitteln im Verhältnis von 0,05 bis etwa 0,5 Teilen zu etwa 1 Teil der bekannten Mittel zu verabreichen. Die pharmazeutische Zusammensetzung kann in Substanz oder als wässrige Lösung vorliegen zusammen mit anderen Materialen wie Konservierungsmitteln, Puffersubstanzen, Mitteln die zur Einstellung der Osmolarität der Lösung vorgesehen sind etc.

Bevorzugt wird das pharmazeutische Mittel auch als Vakzine nach der Etablierung eines Tumors oder als vorbeugende Impfung eingesetzt. Die Impfung erfolgt vorteilhafterweise so, dass es nach der Applikation im Organismus zur Entwicklung eines Schutzes gegen die Ausbreitung oder die Bildung von Tumoren kommt. Selbstverständlich ist es auch möglich, dass die Impfung unmittelbar vor oder zeitnah nach der Manifestation eines. Tumors erfolgt oder als Therapie mehrfach appliziert wird. Dem Fachmann ist bekannt, dass die Behandlung eines Tumors zu nahezu jedem Zeitpunkt auch nach der Bildung von Metastasen von Vorteil sein kann, so dass eine Impfung im Sinne der Erfindung auch eine Applikation des erfindungsgemäßen pharmazeutischen Mittels Wochen, Monate, Jahre bzw. Jahrzehnte nach der Bildung eines Tumors sein kann.

Die Erfindung betrifft auch einen Kit und dessen Verwendung in der Medizin. Es ist bevorzugt, die erfindungsgemäßen Verbindungen oder den Kit, der diese umfasst, in einer Kombinationstherapie, insbesondere zur Behandlung von Tumoren, zu verwenden. Besonders bevorzugt ist hierbei, dass die Kombinationstherapie eine Chemotherapie, eine Zytostatikabehandlung und/oder eine Strahlentherapie umfasst. In einer besonders bevorzugten Ausführungsform der Erfindung ist die Kombinationstherapie eine adjuvante biologisch-spezifizierte Therapieform. Ganz besonders bevorzugt ist hierbei, dass diese Therapieform eine Immuntherapie ist. Weiterhin ist besonders bevorzugt, dass die Kombinationstherapie eine Gentherapie und/oder eine Therapie mit einer erfindungsgemäßen Verbindung umfasst. Dem Fachmann sind verschiedene Kombinationstherapien, insbesondere zur Behandlung von Tumoren, bekannt. Es kann zum Beispiel vorgesehen sein, dass innerhalb einer Kombinationstherapie eine Zytostatikabehandlung erfolgt oder beispielsweise eine Bestrahlung eines bestimmten Tumorareals, wobei diese Behandlung mit einer Gentherapie kombiniert wird, wobei die erfindungsgemäßen Verbindungen als Antikrebsmittel eingesetzt werden. Demgemäß kann es ganz besonders bevorzugt sein, dass die erfindungsgemäßen Verbindungen zur Erhöhung der Sensitivität von Tumorzellen gegenüber Zytostatika und/oder Strahlen verwendet werden. Weiterhin ist es bevorzugt, dass die erfindungsgemäßen Verbindungen zur Hemmung der Vitalität, der Proliferationsrate von Zellen und/oder zur Induktion von Apoptose und eines Zellzyklus-Arrests verwendet werden.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchiai-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-Zell-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute mor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantationsbezogene Malignitäten, metastasierte Tumoren umfassend Gehirnmetastasen, Lungenmetastasen, Lebernetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

In einer weiteren bevorzugten Ausführungsform ist der Tumor ein Kolonkarzinom, ein Magenkarzinom, ein Pankreaskarzinom, ein Dünndarmkrebs, ein Ovarialkarzinom, ein Zervikalkarzinom, ein Lungenkrebs, ein Prostatakrebs, ein Mammakarzinom, ein Nierenzellkarzinom, ein Hirntumor, ein Kopf-Halstumor, ein Leberkarzinom und/oder eine Metastase dieser Tumoren.

In einer weiteren bevorzugten Ausführungsform ist der solide Tumor ein Mamma-, Bronchial-, Kolorektal-und/oder Prostatakarzinom und/oder eine Metastase dieser Tumoren.

In einer weiteren bevorzugten Ausführungsform ist der Tumor des Urogenitaltraktes ein Harnblasenkarzinom und/oder eine Metastase dieser Tumoren.

In einer weiteren bevorzugten Ausführungsform ist die Verlaufskontrolle eine Überwachung der Wirksamkeit einer Antitumorbehandlung.

In einer weiteren bevorzugten Ausführungsform wird mindestens ein erfindungsgemäßes Chelidonin-Derivat und/oder ein erfindungsgemäßes pharmazeutisches Mittel zur Prophylaxe, Prävention, Diagnose, Verminderung, Therapie, Verlaufskontrolle und/oder Nachbehandlung einer Metastasierung, einer Invasion und/oder einer Angiogenese eingesetzt.

In einer weiteren bevorzugten Ausführungsform ist diese Verlaufskontrolle eine Überwachung der Wirksamkeit einer Antitumorbehandlung.

In einer weiteren bevorzugten Ausführungsform wird mindestens ein erfindungsgemäßes Chelidonin-Derivat und/oder ein erfindungsgemäßes pharmazeutisches Mittel in einer Kombinationstherapie verwendet.

In einer weiteren bevorzugten Ausführungsform umfasst diese Kombinationstherapie eine Chemotherapie, eine Zytostatikabehandlung und/oder eine Strahlentherapie.

In einer weiteren bevorzugten Ausführungsform umfasst die Kombinationstherapie eine adjuvante biologisch-spezifizierte Therapieform.

In einer weiteren bevorzugten Ausführungsform ist die Therapieform eine Immuntherapie.

In einer weiteren bevorzugten Ausführungsform wird mindestens ein erfindungsgemäßes Chelidonin-Derivat und/oder ein erfindungsgemäßes pharmazeutisches Mittel zur Erhöhung der Sensitivität von Tumorzellen gegenüber Zytostatika und/oder Strahlen verwendet.

In einer weiteren bevorzugten Ausführungsform wird mindestens ein erfindungsgemäßes Chelidonin-Derivat und/oder ein erfindungsgemäßes pharmazeutisches Mittel zur Hemmung der Vitalität, der Proliferationsrate von Zellen, zur Induktion von Apoptose und/oder eines Zellzyklus-Arrests verwendet.

In einer weiteren bevorzugten Ausführungsform werden mindestens ein erfindungsgemäßes Chelidonin-Derivat und/oder ein erfindungsgemäßes pharmazeutisches Mittel als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und angewendet.

In einer weiteren bevorzugten Ausführungsform liegen mindestens ein erfindungsgemäßes Chelidonin-Derivat und/oder ein erfindungsgemäßes pharmazeutisches Mittel in einer Konzentration von 0,1 bis 99,5, bevorzugt von 0,5 bis 95,0, besonders bevorzugt von 20,0 bis 80,0 Gewichtsprozent in einer Zubereitung vor.

In einer weiteren bevorzugten Ausführungsform wird die Zubereitung oral, subkutan, intravenös, intramuskulär, intraperitoneal und/oder topisch gesetzt.

In einer weiteren bevorzugten Ausführungsform werden mindestens ein erfindungsgemäßes Chelidonin-Derivat und/oder ein erfindungsgemäßes pharmazeutisches Mittel in Gesamtmengen von 0,05 bis 500 mg pro kg, bevorzugt von 5 bis 100 mg pro kg Körpergewicht, je 24 Stunden eingesetzt.

In einer weiteren bevorzugten Ausführungsform werden die erfindungsgemäßen Chelidonin-Derivate dadurch hergestellt, dass Chelidonin-Acetat durch Umsetzung von Chelidonin mit Pyridin und Acetanhydrid erhalten wird.

In einer weiteren bevorzugten Ausführungsform werden die erfindungsgemäßen Chelidonin-Derivate dadurch hergestellt, dass eine Mischung aus Chelidonin, Pyridin und Acetanhydrid bei Raumtemperatur für mindestens 12 Stunden inkubiert wird und folgend diese Mischung in Eiswasser gegossen wird, wobei ein Rohprodukt ausfällt und das Rohprodukt mit Ether extrahiert wird.

In einer weiteren bevorzugten Ausführungsform werden die erfindungsgemäßen Chelidonin-Derivate dadurch hergestellt, dass Trifluoressigsäure-Chelidonylester, Chelydonyl-Tri-chlormethyl-Kohlensäureester und/oder Bernsteinsäure-Chelidoninyl-Methylester durch Umsetzung von Chelidonin in Chloroform mit dem jeweiligen Säurechlorid erhalten werden, wobei dem Gemisch aus Chelidonin, Chloroform und dem jeweiligen Säurechlorid Pyridin zugesetzt wird und die so erhaltene Mischung bei Raumtemperatur für mindestens 4 Stunden inkubiert wird.

In einer weiteren bevorzugten Ausführungsform werden die erfindungsgemäßen Chelidonin-Derivate dadurch hergestellt, dass Chelidonyl-Ethyl-Oxalsäurediester durch die Umsetzung von Chelidonin-Monohydrat und Oxalsäureesterchlorid gewonnen wird.

In einer weiteren bevorzugten Ausführungsform werden die erfindungsgemäßen Chelidonin-Derivate dadurch hergestellt, dass N-(3-Trifluormethylphenyl)-chelidonyl-urethan durch die Umsetzung von Chelidonin-Monohydrat mit 3-Trifluor-methylphenylisocyanat gewonnen wird.

In einer weiteren bevorzugten Ausführungsform werden die erfindungsgemäßen Chelidonin-Derivate dadurch hergestellt, dass Chelidonyl-phenylalanylester durch die Umsetzung von Chelidonin-Monohydrat mit N-(9-Fluorenylmethoxy-carbonyl) -L-phenylalanin gewonnen wird.

In einer weiteren bevorzugten Ausführungsform werden die erfindungsgemäßen Chelidonin-Derivate dadurch hergestellt, dass Chelidonyl-prolylester durch die Umsetzung von Chelidonin-Monohydrat mit N-(9-Fluorenylmethoxy-carbonyl)-L-prolin gewonnen wird.

In einer weiteren bevorzugten Ausführungsform werden die erfindungsgemäßen Chelidonin-Derivate dadurch hergestellt, dass Chelidonyl-analylester durch die Umsetzung von Chelidonin-Monohydrat mit N-(9-Fluorenylmethoxy-carbonyl)-L-alanin gewonnen wird.

In einer weiteren bevorzugten Ausführungsform wird eine Tumorerkrankung dadurch behandelt, dass mindestens ein erfindungsgemäßes Chelidonin-Derivat und/oder ein erfindungsgemäßes pharmazeutisches Mittel mit einem Organismus, bevorzugt einem Menschen oder einem Tier, in Kontakt gebracht werden.

In einer weiteren bevorzugten Ausführungsform erfolgt dieses In-Kontakt-Bringen oral, über Injektion, topisch, vaginal, rektal und/oder nasal.

In einer weiteren bevorzugten Ausführungsform wird ein pharmazeutisches Mittel zur Behandlung von Tumorerkrankungen dadurch hergestellt, dass mindestens ein erfindungsgemäßes Chelidonin-Derivat und/oder ein erfindungsgemäßes pharmazeutisches Mittel mit einem pharmazeutisch verträglichen Träger eingesetzt werden.

Eine weitere bevorzugte Ausführungsform umfasst einen Kit, welcher mindestens ein erfindungsgemäßes Chelidonin-Derivat und/oder ein erfindungsgemäßes pharmazeutisches Mittel gegebenenfalls mit einer Information zum Kombinieren der Inhalte des Kits umfasst.

In einer weiteren bevorzugten Ausführungsform wird dieser Kit zur Prophylaxe oder Therapie von Tumorerkrankungen verwendet. das zurückbleibende Rohprodukt aus Ethanol umkristallisiert.
Ausbeute: 0,9 g (ca. 85 % d. T.)

### 2. Herstellung der Chelidoninester

### Allgemeine Vorschrift:

300 mg Chelidonin werden in 30 ml trockenem Chloroform gelöst und die 1,2-fach molare Menge des jeweiligen Säurechlorids hinzugefügt.
Nach Zugabe von 3 ml Pyridin lässt man das Reaktionsgemisch bei Raumtemperatur über Nacht stehen.

### Aufarbeitung/Reinigung:

Nach Hinzufügen weiterer 100 ml Chloroform wird die organische Phase 4- bis 5-mal mit Wasser gewaschen. Das Lösungsmittel wird am Rotationsverdampfer entfernt und das zurückbleibende Rohprodukt 1 - bis 2-mal aus Ethanol umkristallisiert (Rekristallisation - -20°C).

### 2.1. Herstellung von Trifluoressigsäure-chelidoninylester (A101)

Ansatz: 300 mg Chelidonin, 380 mg Trifluoressigsäureanhydrid
Ausbeute: 300 mg (81 % d. Th.) Fp = 140 - 42 °C

### 2.2. Herstellung von Chelidoninyl-trichlormethyl-kohlensäureester (A102)

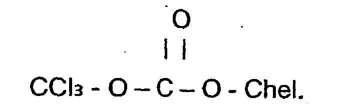

Ansatz: 300 mg Chelidonin, 270 mg Trichlormethylchlorformiat
Ausbeute: 200 mg (48,1 % d. Th.) Fp = 154 - 57°C

### 2.3 Herstellung von Bernsteinsäure-chelidoninyl-methyl-ester (A103)

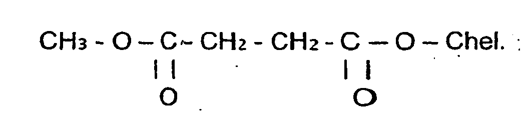

Ansatz: 300 mg Chelidonin, 300 mg Bernsteinsäuremethylesterchlorid
Ausbeute: 200 mg (52,9 % d. Th.) Fp = 84 - 85 °C

### 3. Herstellung von Chelidonyl-ethyl-oxalsäurediester

### 3.1 allgemeine Vorschriften für die Umsetzung mit Carbonsäurechloriden und Anhydriden

- Chelidonin-Monohydrat und die doppelt molare Menge des entsprechenden Carbonsäurechlorids oder Anhydrids (mit 10 % Überschuss) werden in einen Erlenmeyerkolben eingewogen
- ca. 10 ml Pyridin zugeben
- Reaktionsansatz schütteln und 3 Tage bei Raumtemperatur stehen lassen
- Gemisch in einen Scheidetrichter geben und ca. 20 ml Ether hinzugeben, anschließend 5- bis 6-mal mit Wasser waschen
- Lösungsmittel abdampfen
- Reinigung

### Reaktion:

Umsetzung von Chelidonin-Monohydrat (Fluka Ch:425201/1) mit Oxalsäureesterchlorid (Lancaster) 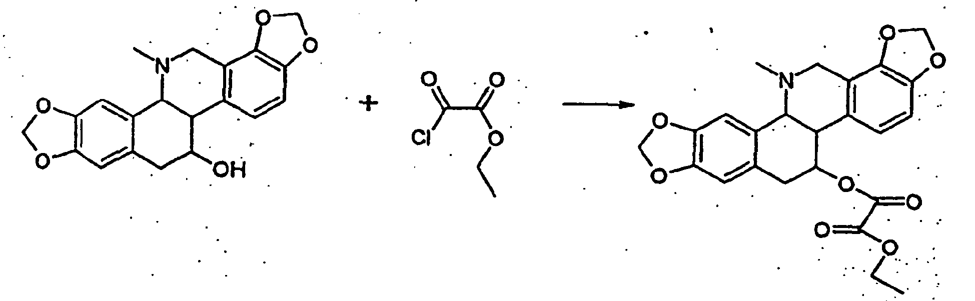 Chelidonin-Monohydrat: M=371,39 g/mol
Oxalsäureesterchlorid: M=136,53 g/mol
Chelidonyl-ethyl-oxalsäurediester: M= 453,42 g/mol, C₂₄H₂₃NO₈
Ausbeute: 25,9 % (80, 4 mg)
- Einwaage: 0,3106 g Chelidonin-Monohydrat und 0,2805 g Oxalsäureesterchlorid
- Flash-Chromatographie (1 % Triethylamin in einer Heptan-Ether-Mischung 8 : 2 v/v)

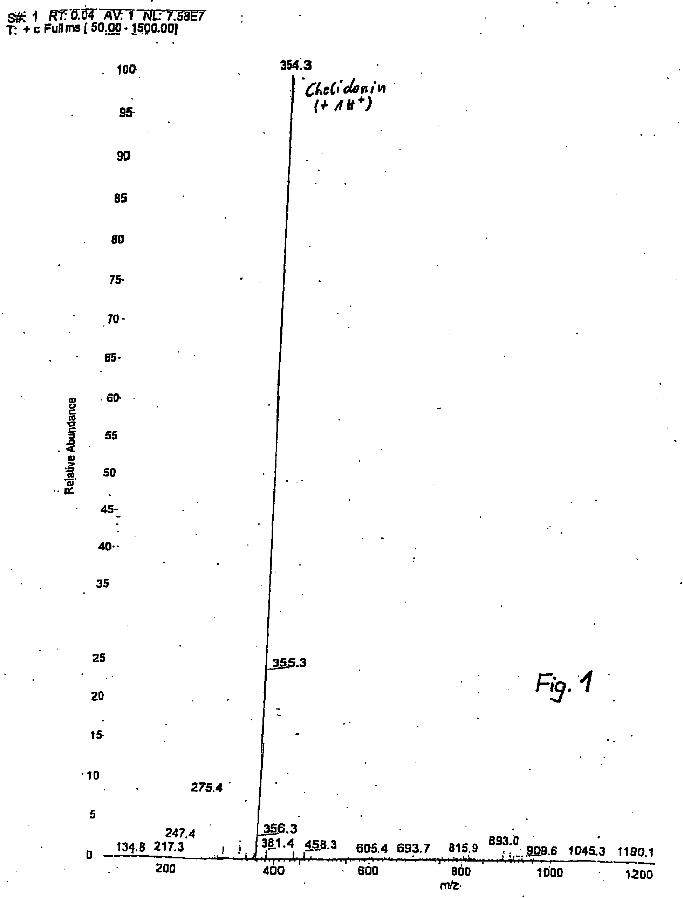

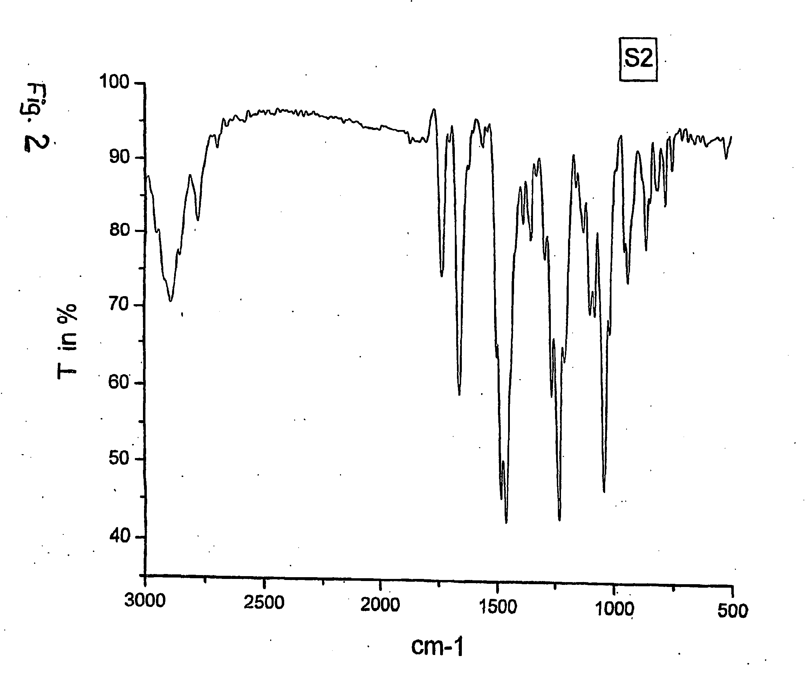

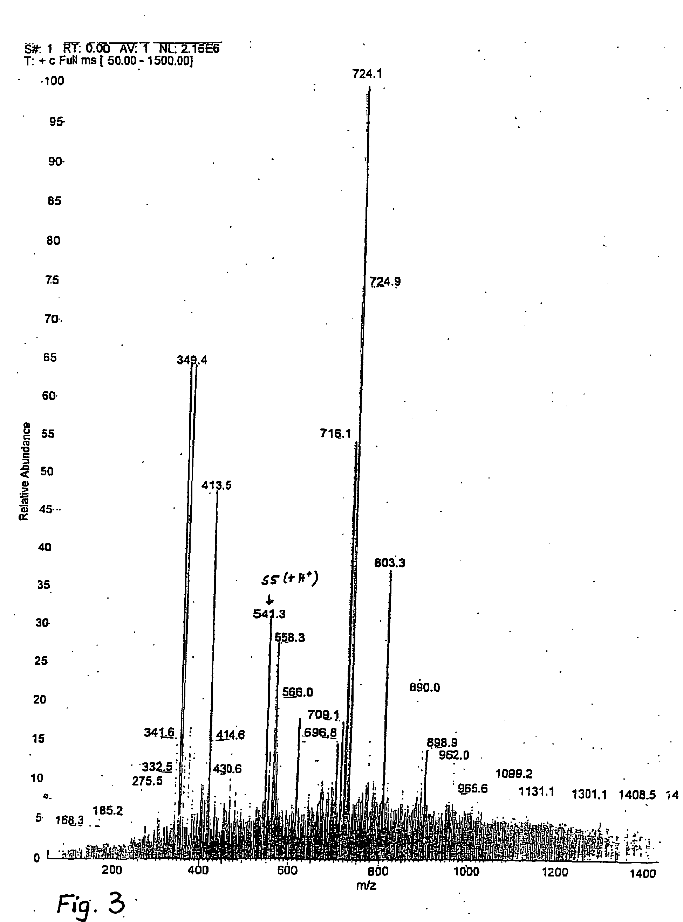

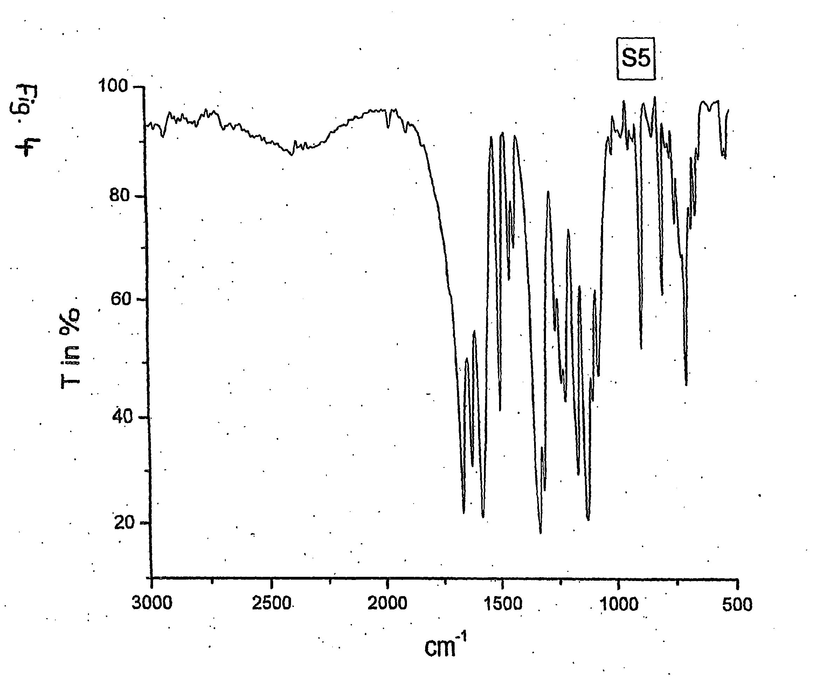

Die Umsetzung und die Herstellung von Chelidonyl-ethyl-oxalsäurediester (S2) ist im IR-Spektrum und im MS-Spektrum gezeigt (Fig. 1 und 2).

### 4. Die Herstellung von N-(3-Trifluormethylphenyl)-chelidonyl-urethan

### 4.1 Allgemeine Vorschriften für das Umsetzen mit Isocyanaten und Isothiocyanaten

- Chelidonin-Monohydrat und die doppelt molare Menge des entsprechenden Isocyanates oder Isothiocyanates (mit 10 % Überschuss) werden in einen Einhalskolben eingewogen
- ca. 40 ml Acetonitril zugeben
- Reaktionsansatz 4 h unter Rückfluss kochen
- Lösungsmittel abdampfen
- Reinigung

### Reaktion:

Umsetzung von Chelidonin-Monohydrat (Fluka Ch:425201/1) mit 3-Trifluormethyl-phenylisocyanat (Riedel-de Haën AG) 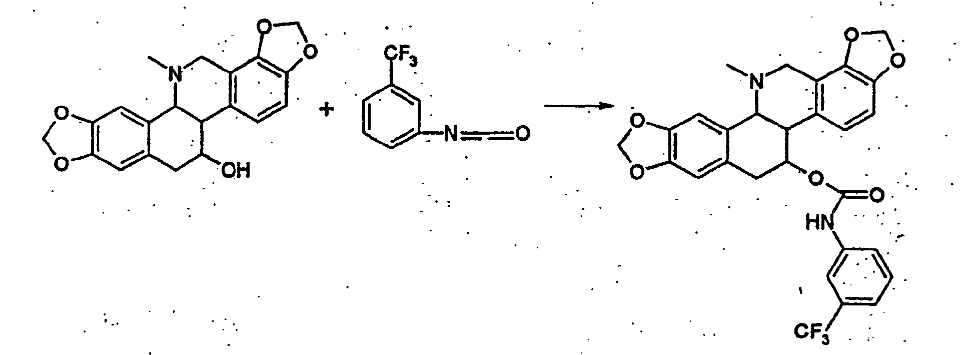 Chelidonin-Monohydrat: M = 371,39 g/mol
3-Trifluormethylphenylisocyanat : M = 187,11 g/mol
N- (3-Trifluormethylphenyl) -chelidonyl-urethan:
M = 540,47 g/mol, C₂₈H₂₃F₃N₂O₆
Ausbeute: 34,8 % (104,4 mg)

### 5.1 Allgemeine Vorschriften für die Umsetzung mit FMOC-geschützten Aminosäuren

- Chelidonin-Monohydrat, die äquimolare Menge der FMOC-geschützten Aminosäure und die doppelt molare Menge Dicyclohexylcarbodiimid (mit 10 % Überschuss) werden in einen Einhalskolben eingewogen
- ca. 50 ml Essigester zugeben
- Reaktionsansatz 3 Tage Rühren bei Raumtemperatur Essigester abdampfen
- Entfernen des Dicyclohexylharnstoffes: Tetrachlorkohlenstoff zugeben, erwärmen und heiß filtrieren (Rückstand ist Dicyclohexylharnstoff)
- Tetrachlorkohlenstoff abdampfen
- Entfernen der Schutzgruppe: ca. 4 ml Morpholin hinzugeben und 30 min stehen lassen
- Morpholin abdampfen
- Reinigung

### Reaktion:

Umsetzung von Chelidonin-Monohydrat (Fluka Ch:425201/1) mit N-(9-Fluorenylmethoxycarbonyl)-L-phenylalanin (Aldrich) 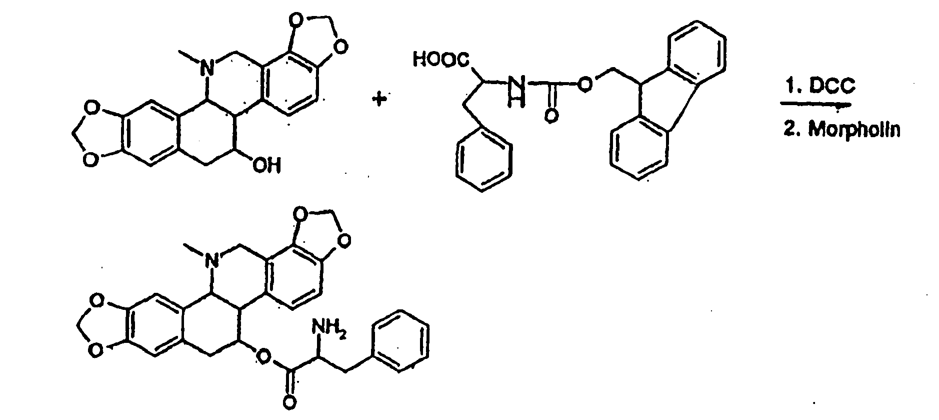 Chelidonin-Monohydrat: M = 371,39 g/mol
N-(9-Fluorenylmethoxycarbonyl)-L-phenylalanin:
M = 387,44 g/mol
Dicyclohexylcarbodiimid (DCC): M = 206,33 g/mol Chelidonyl-phenylalanylester: M = 500,52 g/mol, C₂₉H₂₈N₂O₆
Ausbeute: 41,1 % (113,9 mg)
- Einwaage: 0,277 g Chelidonin-Monohydrat, 0,2902 g N-(9-Fluorenylmethoxycarbonyl)-L-phenylalanin und 0,3662 g Dicyclohexylcarbodiimid
- Flash-Chromatographie (1'% Triethylamin in einer Heptan-Essigester-Mischung 7 : 3 v/v)

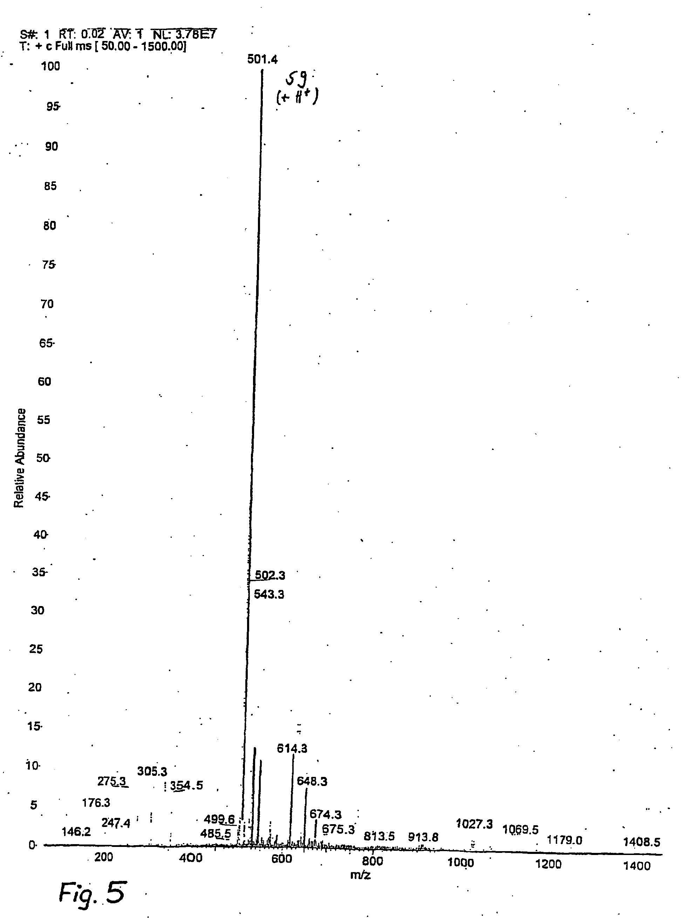

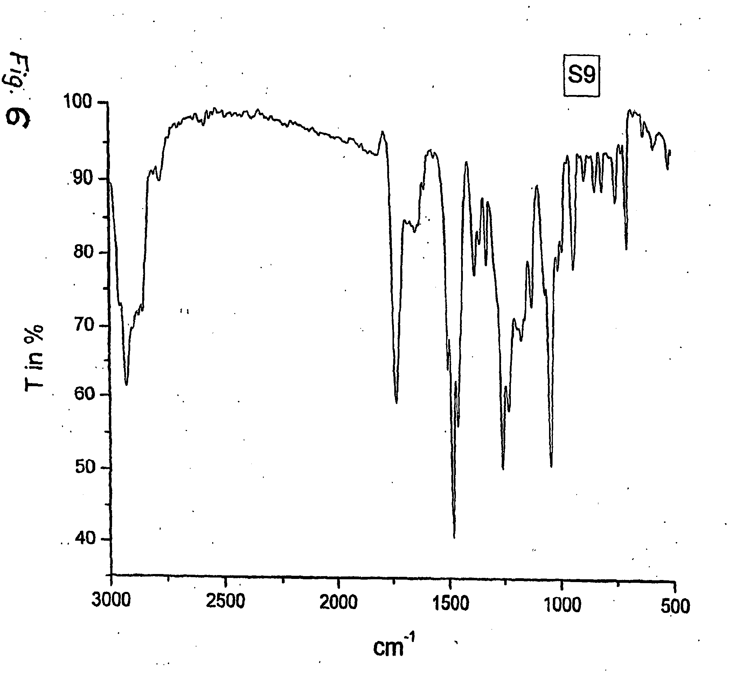

Die Herstellung von Chelidonyl-phenylalanylester (S9) wurde anhand der IR- und MS-Spektren gezeigt (Fig. 5 und 6).

### 6. Herstellung von Chelidonyl-prolylester

### 6.1 siehe allgemeine Vorschriften für die Umsetzung mit FMOC-geschützten Aminosäuren

### Reaktion:

Umsetzung von Chelidonin-Monohydrat (Fluka Ch:425201/1) mit N-(9-Fluorenylmethoxycarbonyl)-L-prolin (Aldrich) 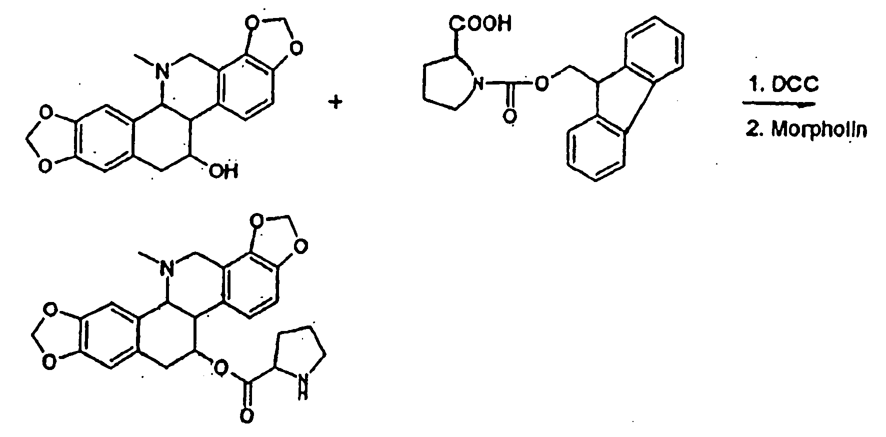 Chelidonin-Monohydrat: M = 371,39 g/mol
N-(9-Fluorenylmethoxycarbonyl)-L-prolin: M = 337,38 g/mol
Dicyclohexylcarbodiimid (DCC): M = 206,33 g/mol
Chelidonyl-prolylester: M = 450,47 g/mol, C₂₅H₂₆N₂O₆
Ausbeute: 36,3 % (101,3 mg)
- Einwaage: 0,2789 g Chelidonin-Monohydrat, 0,2790 g N-(9-Fluorenylmethoxycarbonyl)-L-prolin und 0,3705 g Dicyclohexylcarbodiimid
- Flash-Chromatographie (1 % Triethylamin in einer Heptan-Essigester-Mischung 7 : 3 v/v)

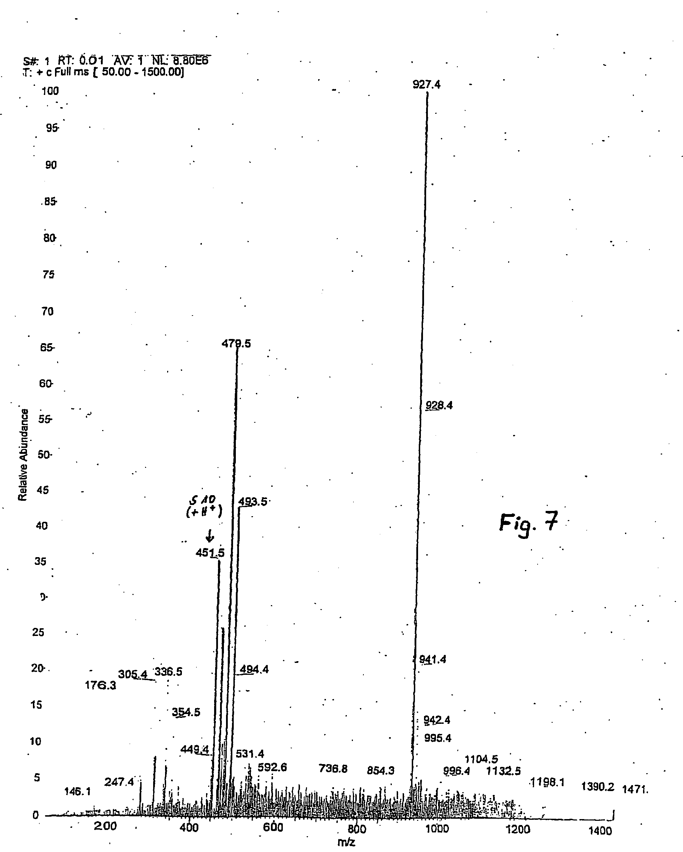

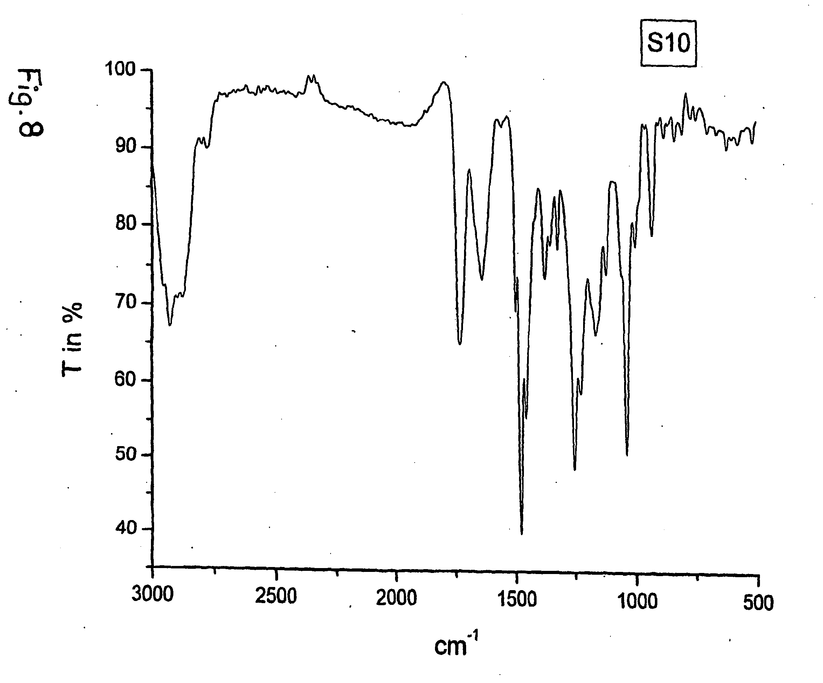

Die Herstellung von Chelidonyl-prolylester (S10) wurde anhand der IR- und MS-Spektren gezeigt (Fig. 7 und 8).

### 7. Herstellung von Chelidonyl-alanylester

### 7.1 siehe allgemeine Vorschriften für die Umsetzung mit FMOC-geschützten Aminosäuren

### Reaktion:

Umsetzung von Chelidonin-Monohydrat (Fluka Ch:425201/1) mit N-(9-Fluorenylmethoxycarbonyl)-L-alanin (Aldrich) 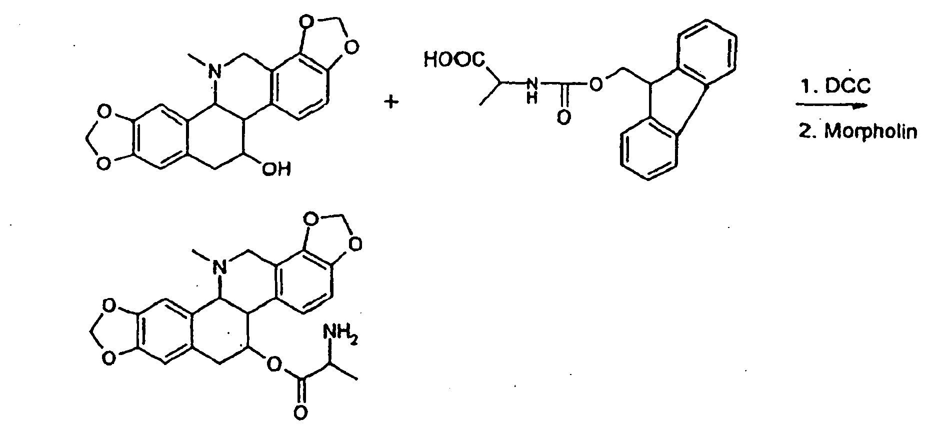 Chelidonin-Monohydrat: M = 371,39 g/mol
N-(9-Fluorenylmethoxycarbonyl)-L-alanin: M = 311,32 g/mol
Dicyclohexylcarbodiimid (DCC): M = 206,33 g/mol
Chelidonyl-analylester: M = 424,43 g/mol, C₂₃H₂₄N₂O₆
Ausbeute: 33,5 % (101,0 mg)
- Einwaage: 0,3015 g Chelidonin-Monohydrat, 0,2660 g N-(9-Fluorenylmethoxycarbonyl)-L-alanin und 0,3698 g Dicyclohexylcarbodiimid
- Flash-Chromatographie (1 % Triethylamin in einer Heptan-Essigester-Mischung 7 : 3 v/v)

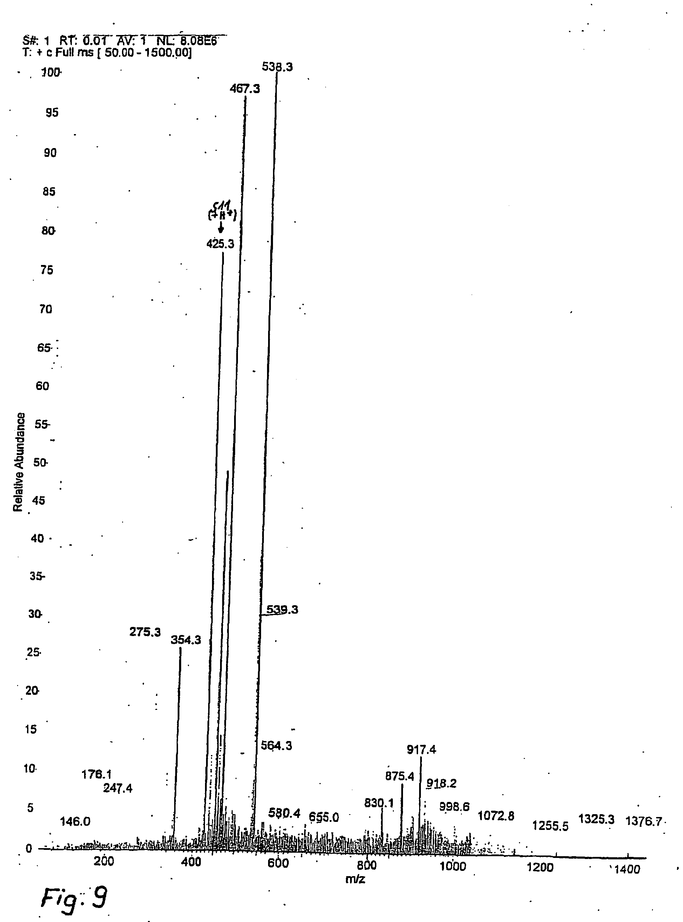

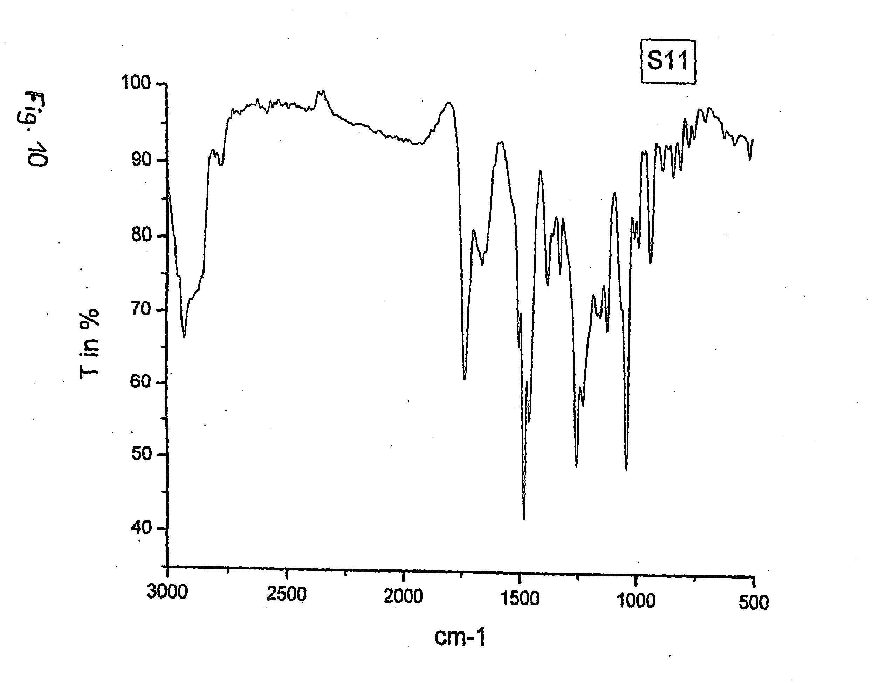

Die Herstellung von Chelidonyl-alanylester (S11) wurde anhand der IR- und MS-Spektren gezeigt (Fig. 9 und 10).

Die folgenden Resultate wurden mit einem MTT-Zytotoxizitätstest gewonnen. Die Werte sind IC50-Werte in ng/ml. Es wird gezeigt, dass Chelidoninyl-trichlormethyl-kohlensäureester eine ähnliche Aktivität wie natives Chelidonin hat; Trifluoressigsäure-chelidoninyester und Bernsteinsäure-chelidoninyl-methyl-ester zeigen eine 5-ester eine ähnliche Aktivität wie natives Chelidonin hat; Trifluoressigsäure-chelidoninyester und Bernsteinsäure-chelidoninyl-methyl-ester zeigen eine 5-fache (im Bereich zwischen 4 - 10-fach) und ungefähr 30-fach (im Bereich von 50 - 150-fach) höhere Aktivität zu dem nativen Chelidonin.

| | Chelidonine | Trifluoressigsäure-chelidoninyl-ester | Cheli-doninyl-trichlormethyl-kohlensäure-ester | Bernsteinsäure-chelidoninyl-methyl-ester |
|---|---|---|---|---|
| T47D (breast) | 200 | 20 | 200 | 20 |
| Colo205 (colon) | 370 | 45 | 500 | 3.5 |
| Panc1 (pancreas) | 250 | 60 | 250 | 5 |
| BxPC3 (pancreas) | > 8000 | 30 | > 8000 | < 3.5 |
| U373MG (astrocyt) | > 8000 | > 4000 | > 8000 | 4000 |
| SK-OV3 (ovarian) | nicht getestet | 60 | nicht getestet | 3.5 |

## Patentansprüche

1. Neue Chelidonin-Derivate mit einer antitumoralen Wirkung ausgewählt aus der Gruppe umfassend Trifluoressigsäure-Chelidonylester, Chelidonyl-trichlormethylkohlensäureester, Bernsteinsäure-chelidoninyl-methylester, Chelidonyl-ethyloxalsäure-diester, N-(3-Trifluormethylphenyl)-chelidonyl-urethan, Chelidonyl-phenylalanylester, Chelidonyl-prolylester und/oder Chelidonyl-alanylester.

2. Chelidonin-Derivate nach Anspruch 1,
**dadurch gekennzeichnet, dass**
die antitumorale Wirkung eine Modulation eines Zellwachstums, einer Zelldifferenzierung und/oder einer Zellteilung ist.

3. Pharmazeutisches Mittel umfassend mindestens ein Chelidonin-Derivat gemäß Anspruch 1 oder 2 gegebenenfalls zusammen mit einem verträglichen pharmazeutischen Träger, Adjuvants und/oder Vehikel.

4. Pharmazeutisches Mittel nach Anspruch 3,
**dadurch gekennzeichnet, dass**
die Träger Liposomen, Siosomen und/oder Niosomen sind.

5. Verwendung eines Chelidonin-Derivates ausgewählt aus der Gruppe umfassend Chelidonin-Acetat, Trifluoressigsäure-Chelidonylester, Chelidonyltrichlormethylkohlensäureester, Bernsteinsäurechelidoninyl-methylester, Chelidonyl-ethyl-oxalsäurediester, N-(3-Trifluormethylphenyl)-chelidonyl-urethan, Chelidonyl-phenylalanylester, Chelidonyl-prolylester und/oder Chelidonyl-alanylester zur Herstellung eines Arzneimittels zur Prophylaxe, Therapie, Verlaufskontrolle und/oder Nachbehandlung von mit Zellwachstum, -differenzierung und/oder -teilung im Zusammenhang stehenden Krankheiten.

6. Verwendung nach dem vorhergehenden Anspruch,
**dadurch gekennzeichnet, dass**
die Krankheit ein Tumor ist.

7. Verwendung nach dem vorhergehenden Anspruch,
**dadurch gekennzeichnet, dass**
die Tumorerkrankungen ausgewählt sind aus der Gruppe der neoplastischen Tumoren, der endzündlichen Tumoren und/oder der Abzesse, Ergüsse und Ödeme.

8. Verwendung nach einem der vorhergehenden Ansprüche,
**dadurch gekennzeichnet, dass**
mindestens ein Chelidonin-Derivat gemäß Anspruch 1 oder 2 und/oder ein pharmazeutisches Mittel gemäß einem der Ansprüche 3 bis 4 zur Herstellung eines Arzneimittels zur Prophylaxe, Prävention, Diagnose, Verminderung, Therapie, Verlaufskontrolle und/oder Nachbehandlung einer Metastasierung, einer Invasion und/oder einer Angiogenese eingesetzt werden.

9. Verwendung nach einem der vorhergehenden Ansprüche,
**dadurch gekennzeichnet, dass**
die Verlaufskontrolle eine Überwachung der Wirksamkeit einer Antitumorbehandlung ist.

10. Verwendung nach einem der vorhergehenden Ansprüche,
**dadurch gekennzeichnet, dass**
mindestens ein Chelidonin-Derivat gemäß Anspruch 1 oder 2 und/oder ein pharmazeutisches Mittel gemäß einem der Ansprüche 3 bis 4 in einer Kombinationstherapie verwendet werden.

11. Verwendung nach dem vorhergehenden Anspruch,
**dadurch gekennzeichnet, dass**
die Kombinationstherapie eine Chemotherapie, eine Zytostatikabehandlung und/oder eine Strahlentherapie umfasst.

12. Verwendung nach dem vorhergehenden Anspruch,
**dadurch gekennzeichnet, dass**
die Kombinationstherapie eine adjuvante biologisch-spezifizierte Therapieform umfasst.

13. Verwendung nach dem vorhergehenden Anspruch,
**dadurch gekennzeichnet, dass**
die Therapieform eine Immuntherapie ist.

14. Verwendung nach einem der vorhergehenden Ansprüche zur Erhöhung der Sensitivität von Tumorzellen gegenüber Zytostatika und/oder Strahlen.

15. Verwendung nach einem der vorhergehenden Ansprüche zur Herstellung eines Arzneimittels zur Hemmung der Vitalität der Proliferationsrate von Zellen, zur Induktion von Apoptose und/oder eines Zellzyklus-Arrests.

16. Verwendung nach, dem vorhergehenden Anspruch,
**dadurch gekennzeichnet, dass**
mindestens ein Chelidonin-Derivat gemäß Anspruch 1 oder 2 und/oder ein pharmazeutisches Mittel gemäß einem der Ansprüche 3 bis 4 in einer Konzentration von 0,1 bis 99,5, bevorzugt von 0,5 bis 95,0, besonders bevorzugt von 20,0 bis 80.0 Gewichtsprozent in einer Zubereitung vorliegen.

17. Verwendung nach einem der vorhergehenden Ansprüche,
**dadurch gekennzeichnet, dass**
mindestens ein Chelidonin-Derivat gemäß Anspruch 1 oder 2 und/oder ein pharmazeutisches Mittel gemäß einem der Ansprüche 3 bis 4 in Gesamtmengen von 0,05 bis 500 mg pro kg, bevorzugt von 5 bis 100 mg pro kg Körpergewicht, bevorzugt je 24 Stunden eingesetzt werden.

18. Verfahren zur Herstellung der Chelidonin-Derivate gemäß Anspruch 1 oder 2,
**dadurch gekennzeichnet, dass**
Chelidonin-Acetat durch Umsetzung von Chelidonin mit Pyridin und Acetanhydrid erhalten wird.

19. Verfahren nach dem vorhergehenden Anspruch,
**dadurch gekennzeichnet, dass**
eine Mischung aus Chelidonin, Pyridin und Acetanhydrid bei Raumtemperatur für mindestens 12 Stunden inkubiert wird und folgend diese Mischung in Eiswasser gegossen wird, wobei ein Rohprodukt ausfällt und das Rohprodukt mit Ether extrahiert wird.

20. Verfahren nach einem der vorhergehenden Ansprüche,
**dadurch gekennzeichnet, dass**
Trifluoressigsäure-Chelidonylester, Chelydonyl-Trichlormethyl-Kohlensäureester und/oder Bernsteinsäure-Chelidoninyl-Methylester durch Umsetzung von Chelidonin mit Chloroform und dem jeweiligen Säurechlorid erhalten werden, wobei dem Gemisch aus Chelidonin, Chloroform und dem jeweiligen Säurechlorid Pyridin zugesetzt wird und die so erhaltene Mischung bei Raumtemperatur für mindestens 4 Stunden inkubiert wird.

21. Verfahren nach einem der vorhergehenden Ansprüche,
**dadurch gekennzeichnet, dass**
Chelidonyl-Ethyl-Oxalsäurediester durch die Umsetzung von Chelidonin-Monophosphat und Oxalsäureesterchlorid gewonnen wird.

22. Verfahren nach einem der vorhergehenden Ansprüche,
**dadurch gekennzeichnet, dass**
N- (3-Trifluormethylphenyl) -chelidonyl-urethan durch die Umsetzung von Chelidonin-Monohydrat mit 3-Trifluormethylphenylisocyanat gewonnen wird.

23. Verfahren nach einem der vorhergehenden Ansprüche,
**dadurch gekennzeichnet, dass**
Chelidonyl-phenylalanylester durch die Umsetzung von Chelidonin-Monohydrat mit N-(9-Fluorenylmethoxycarbonyl) -L-phenylalanin gewonnen wird.

24. Verfahren nach einem der vorhergehenden Ansprüche,
**dadurch gekennzeichnet, dass**
Chelidonyl-prolylester durch die Umsetzung von Chelidonin-Monohydrat mit N- (9-Fluorenylmethoxycarbonyl) -L-prolin gewonnen wird.

25. Verfahren nach einem der vorhergehenden Ansprüche,
**dadurch gekennzeichnet, dass**
Chelidonyl-analylester durch die Umsetzung von Chelidonin-Monohydrat mit N- (9-Fluorenylmethoxycarbonyl) -L-alanin gewonnen wird.

26. Verfahren zur Herstellung eines pharmazeutischen Mittels zur Behandlung einer Tumorerkrankung,
**dadurch gekennzeichnet, dass**
mindestens ein Chelidonin-Derivat gemäß Anspruch 1 oder 2 und/oder ein pharmazeutisches Mittel gemäß einem der Ansprüche 3 bis 4 mit einem pharmazeutisch verträglichen Träger eingesetzt werden.

## Claims

1. New chelidonine derivatives having an anti-tumoral effect, selected from the group comprising chelidoninyl trifluoroacetate, chelidoninyl trichloromethyl carbonate, chelidoninyl methyl succinate, chelidoninyl ethyl oxalate, N-(3-trifluoromethylphenyl)chelidoninylurethane, phenylalanine chelidoninyl ester, proline chelidoninyl ester and/or alanine chelidoninyl ester.

2. The chelidonine derivatives according to claim 1,
**characterized in that**
the anti-tumoral effect is modulation of cell growth, cell differentiation and/or cell division.

3. A pharmaceutical agent comprising at least one chelidonine derivative according to claim 1 or 2, optionally together with a tolerable pharmaceutical carrier, adjuvant and/or vehicle.

4. The pharmaceutical agent according to claim 3,
**characterized in that**
the carriers are liposomes, siosomes and/or niosomes.

5. Use of a chelidonine derivative selected from the group comprising chelidonine acetate, chelidoninyl trifluoroacetate, chelidoninyl trichloromethyl carbonate, chelidoninyl methyl succinate, chelidoninyl ethyl oxalate, N-(3-trifluoromethylphenyl)chelidoninylurethane, phenylalanine chelidoninyl ester, proline chelidoninyl ester and/or alanine chelidoninyl ester in the preparation of a drug for the prophylaxis, therapy, follow-up and/or aftercare of diseases associated with cell growth, cell differentiation and/or cell division.

6. The use according to the preceding claim,
**characterized in that**
the disease is a tumor disease.

7. The use according to the preceding claim,
**characterized in that**
the tumor diseases are selected from the group of neoplastic tumors, inflammatory tumors and/or abscesses, effusions and edema.

8. The use according to any of the preceding claims,
**characterized in that**
at least one chelidonine derivative according to claim 1 or 2 and/or a pharmaceutical agent according to any of claims 3 to 4 are employed in the preparation of a drug for the prophylaxis, prevention, diagnosis, attenuation, therapy, follow-up and/or aftercare of metastase formation, invasion and/or angiogenesis.

9. The use according to any of the preceding claims,
**characterized in that**
said follow-up is monitoring the effectiveness of an anti-tumor treatment.

10. The use according to any of the preceding claims,
**characterized in that**
at least one chelidonine derivative according to claim 1 or 2 and/or a pharmaceutical agent according to any of claims 3 to 4 are employed in a combination therapy.

11. The use according to any of the preceding claims,
**characterized in that**
said combination therapy comprises a chemotherapy, a treatment with cytostatic agents and/or a radiotherapy.

12. The use according to the preceding claim,
**characterized in that**
the combination therapy comprises an adjuvant, biologically specified form of therapy.

13. The use according to the preceding claim,
**characterized in that**
said form of therapy is an immune therapy.

14. The use according to any of the preceding claims to increase the sensitivity of tumor cells to cytostatic agents and/or radiation.

15. The use according to any of the preceding claims in the preparation of a drug for inhibiting the vitality of the proliferation rate of cells, for inducing apoptosis and/or cell cycle arrest.

16. The use according to the preceding claim,
**characterized in that**
at least one chelidonine derivative according to claim 1 or 2 and/or a pharmaceutical agent according to any of claims 3 to 4 are present in a preparation at a concentration of from 0.1 to 99.5, preferably from 0.5 to 95.0, and more preferably from 20.0 to 80.0% by weight.

17. The use according to any of the preceding claims,
**characterized in that**
at least one chelidonine derivative according to claim 1 or 2 and/or a pharmaceutical agent according to any of claims 3 to 4 are employed in overall amounts of from 0.05 to 500 mg per kg, preferably from 5 to 100 mg per kg body weight per 24 hours.

18. A method for the preparation of the chelidonine derivatives according to claim 1 or 2,
**characterized in that**
chelidonine acetate is obtained by reacting chelidonine with pyridine and acetic anhydride.

19. The method according to the preceding claim,
**characterized in that**
a mixture of chelidonine, pyridine and acetic anhydride is incubated for at least 12 hours at room temperature and this mixture is subsequently poured in ice water, so that a raw product precipitates, and the raw product is extracted with ether.

20. The method according to any of the preceding claims,
**characterized in that**
chelidoninyl trifluoroacetate, chelidoninyl trichloromethyl carbonate and/or chelidoninyl methyl succinate are obtained by reacting chelidonine in chloroform with the respective acid chloride, the mixture of chelidonine, chloroform and the respective acid chloride being added with pyridine, and the resulting mixture being incubated for at least 4 hours at room temperature.

21. The method according to any of the preceding claims,
**characterized in that**
chelidoninyl ethyl oxalate is obtained by reacting chelidonine monophosphate with oxalic ester chloride.

22. The method according to any of the preceding claims,
**characterized in that**
N-(3-trifluoromethylphenyl)chelidoninylurethane is obtained by reacting chelidonine monohydrate with 3-trifluoromethylphenyl isocyanate.

23. The method according to any of the preceding claims,
**characterized in that**
phenylalanine chelidoninyl ester is obtained by reacting chelidonine monohydrate with N-(9-fluorenylmethyloxycarbonyl)-L-phenylalanine.

24. The method according to any of the preceding claims,
**characterized in that**
proline chelidoninyl ester is obtained by reacting chelidonine monohydrate with N-(9-fluorenylmethyloxycarbonyl)-L-proline.

25. The method according to any of the preceding claims,
**characterized in that**
alanine chelidoninyl ester is obtained by reacting chelidonine monohydrate with N-(9-fluorenylmethyloxycarbonyl)-L-alanine.

26. A method for the production of a pharmaceutical agent for the treatment of a tumor disease,
**characterized in that**
at least one chelidonine derivative according to claim 1 or 2 and/or a pharmaceutical agent according to any of claims 3 to 4 are employed together with a pharmaceutically tolerable carrier.

## Revendications

1. Nouveaux dérivés de la chélidonine à action antitumorale, sélectionnés parmi le groupe comprenant l'ester chélidonique d'acide trifluoroacétique, l'ester chélidonique d'acide trichlorométhyl-carboxylique, le méthylester chélidonique d'acide succinique, le di-ester éthylchélidonique d'acide oxalique, le N-(3-trifluorométhylphényl)-chélidonyl-uréthane, le phénylalanylester chélidonique, le prolylester chélidonique et/ou l'alanylester chélidonique.

2. Dérivés de la chélidonine selon la revendication 1,
**caractérisés en ce que**
l'action antitumorale est une modulation d'une croissance cellulaire, d'une différentiation cellulaire et/ou d'une division cellulaire.

3. Agent pharmaceutique comprenant au moins un dérivé de la chélidonine selon la revendication 1 ou 2, le cas échéant, conjointement à un support, un adjuvant et/ou un véhicule pharmaceutique compatible.

4. Agent pharmaceutique selon la revendication 3,
**caractérisé en ce que**
les supports sont des liposomes, des siosomes et/ou des niosomes.

5. Utilisation d'un dérivé de la chélidonine sélectionné parmi le groupe comprenant l'acétate de chélidonine, l'ester chélidonique d'acide trifluoroacétique, l'ester chélidonique d'acide trichlorométhyl-carboxylique, le méthylester chélidonique d'acide succinique, le di-ester éthylchélidonique d'acide oxalique, le N-(3-trifluorométhylphényl)-chélidonyl-uréthane, le phénylalanylester chélidonique, le prolylester chélidonique, et/ou l'alanylester chélidonique pour la fabrication d'un médicament en vue de la prophylaxie, de la thérapie, du contrôle de l'évolution et/ou du post-traitement de maladies en rapport avec la croissance cellulaire, la différentiation cellulaire et/ou la division cellulaire.

6. Utilisation selon la revendication précédente,
**caractérisée en ce que**
la maladie est une tumeur.

7. Utilisation selon la revendication précédente,
**caractérisée en ce que**
les maladies tumorales sont sélectionnées parmi le groupe des tumeurs néoplasiques, des tumeurs inflammatoires et/ou des abcès, des épanchements et des oedèmes.

8. Composé selon l'une quelconque des revendications précédentes,
**caractérisé en ce que**
l'on utilise au moins un dérivé la chélidonine selon la revendication 1 ou 2, et/ou un agent pharmaceutique selon l'une quelconque des revendications 3 à 4 pour la fabrication d'un médicament en vue de la prophylaxie, de la prévention, du diagnostic, de l'amoindrissement, de la thérapie, du contrôle de l'évolution et/ou du post-traitement d'une métastase, d'une invasion et/ou d'une angiogenèse.

9. Utilisation selon l'une quelconque des revendications précédentes,
**caractérisée en ce que**
le contrôle de l'évolution est une surveillance de l'efficacité d'un traitement antitumoral.

10. Utilisation selon l'une quelconque des revendications précédentes,
**caractérisée en ce que**
l'on utilise au moins un dérivé de la chélidonine selon la revendication 1 ou 2
et/ou un agent pharmaceutique selon l'une quelconque des revendications 3 à 4 dans une thérapie de combinaison.

11. Utilisation selon l'une quelconque des revendications précédentes,
**caractérisée en ce que**
la thérapie de combinaison comprend une chimiothérapie, un traitement par cytostatiques et/ou une radiothérapie.

12. Utilisation selon la revendication précédente,
**caractérisée en ce que**
la thérapie de combinaison comprend une forme de thérapie adjuvante spécifiée du point de vue biologique.

13. Utilisation selon la revendication précédente,
**caractérisée en ce que**
la forme de thérapie est une immunothérapie.

14. Utilisation selon l'une quelconque des revendications précédentes en vue de l'augmentation de la sensibilité de cellules tumorales vis-à-vis des cytostatiques et/ou des rayons.

15. Utilisation selon l'une quelconque des revendications précédentes pour la fabrication d'un médicament en vue de l'inhibition de la vitalité du taux de prolifération de cellules, en vue de l'induction de l'apoptose et/ou d'un arrêt du cycle cellulaire.

16. Utilisation selon la revendication précédente,
**caractérisée en ce que**
sont présents au moins un dérivé de la chélidonine selon la revendication 1 ou 2, et/ou un agent pharmaceutique selon l'une quelconque des revendications 3 à 4 dans une concentration de 0,1 à 99,5, de préférence de 0,5 à 95,0, de manière particulièrement préférée, de 20,0 à 80,0 pour cent en poids dans une préparation,

17. Utilisation selon l'une quelconque des revendications précédentes,
**caractérisée en ce que**
l'on utilise au moins un dérivé de la chélidonine selon la revendication 1 ou 2
et/ou un agent pharmaceutique selon l'une quelconque des revendications 3 à 4, dans des quantités totales de 0,05 à 500 mg par kg, de préférence de 5 à 100 mg par kg de poids corporel, de préférence toutes les 24 heures.

18. Procédé de préparation de dérivés de la chélidonine selon la revendication 1 ou 2,
**caractérisé en ce que**
l'on obtient l'acétate de chélidonine par réaction de la chélidonine avec la pyridine et l'anhydride acétique.

19. Procédé selon la revendication précédente,
**caractérisé en ce que**
un mélange de chélidonine, de pyridine et d'anhydride acétique est incubé pendant au moins 12 heures à température ambiante et qu'ensuite ce mélange est versé dans de l'eau glacée, un produit brut précipitant et le produit brut étant extrait à l'éther.

20. Procédé selon l'une quelconque des revendications précédentes,
**caractérisé en ce que**
l'ester chélidonique d'acide trifluoroacétique, l'ester chélidonique d'acide trichlorométhyl-carboxylique et/ou le méthylester chélidonique d'acide succinique sont obtenus par réaction de la chélidonine avec du chloroforme et avec le chlorure d'acide correspondant, de la pyridine étant ajoutée au mélange de chélidonine, de chloroforme et du chlorure d'acide correspondant et le mélange ainsi obtenu étant incubé à température ambiante pendant au moins 4 heures.

21. Procédé selon l'une quelconque des revendications précédentes,
**caractérisé en ce que**
le di-ester éthylchélidonique d'acide oxalique est obtenu par réaction du monophosphate de chélidonine et du chlorure d'ester oxalique.

22. Procédé selon l'une quelconque des revendications précédentes,
**caractérisé en ce que**
le N-(3-trifluorométhylphényl)-chélidonyl-uréthane est obtenu par réaction du monohydrate de chélidonine avec l'isocyanate de 3-trifluorométhylphényle.

23. Procédé selon l'une quelconque des revendications précédentes,
**caractérisé en ce que**
le phénylalanylester chélidonique est obtenu par réaction du monohydrate de chélidonine avec la N-(9-fluorénylméthoxycarbonyl)-L-phénylalanine.

24. Procédé selon l'une quelconque des revendications précédentes,
**caractérisé en ce que**
le prolylester chélidonique est obtenu par réaction du monohydrate de chélidonine avec la N-(9-fluorénylméthoxycarbonyl)-L-proline.

25. Procédé selon l'une quelconque des revendications précédentes,
**caractérisé en ce que**
l'alanylester chélidonique est obtenu par réaction du monohydrate de chélidonine avec la N-(9-fluorénylméthoxycarbonyl)-L-alanine.

26. Procédé en vue de la fabrication d'un agent pharmaceutique pour le traitement d'une maladie tumorale,
**caractérisé en ce que**
l'on utilise au moins un dérivé de la chélidonine selon la revendication 1 ou 2 et/ou un agent pharmaceutique selon l'une quelconque des revendications 3 à 4 avec un support compatible du point de vue pharmaceutique.
